(19) Europäisches Patentamt / European Patent Office / Office européen des brevets

(11) EP 3 390 992 B1

(12) **EUROPÄISCHE PATENTSCHRIFT**

(45) Veröffentlichungstag und Bekanntmachung des Hinweises auf die Patenterteilung:
**10.12.2025 Patentblatt 2025/50**

(21) Anmeldenummer: **16822432.7**

(22) Anmeldetag: **16.12.2016**

(51) Internationale Patentklassifikation (IPC):
***G01J 3/46*** *(2006.01)* ***A45D 44/00*** *(2006.01)*
***A61B 5/103*** *(2006.01)* ***G16H 50/30*** *(2018.01)*
***A61B 5/00*** *(2006.01)*

(52) Gemeinsame Patentklassifikation (CPC):
**A45D 44/005; A61B 5/1032; A61B 5/448; A61B 5/7275; G01J 3/462; G16H 50/30;**
A61B 5/7264

(86) Internationale Anmeldenummer:
**PCT/EP2016/081357**

(87) Internationale Veröffentlichungsnummer:
**WO 2017/103050 (22.06.2017 Gazette 2017/25)**

(54) **VERFAHREN UND DATENVERARBEITUNGSVORRICHTUNG ZUM COMPUTERGESTÜTZTEN ERMITTELN VON EIGENSCHAFTEN VON HAARFARBEN**

METHOD AND DATA PROCESSING DEVICE FOR ASCERTAINING PROPERTIES OF HAIR COLORS IN A COMPUTER-ASSISTED MANNER

PROCÉDÉ ET DISPOSITIF DE TRAITEMENT DE DONNÉES POUR DÉTERMINATION ASSISTÉE PAR ORDINATEUR DE CARACTÉRISTIQUES DE COULEURS DE CHEVEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2015 DE 102015225458**

(43) Veröffentlichungstag der Anmeldung:
**24.10.2018 Patentblatt 2018/43**

(73) Patentinhaber: **Henkel AG & Co. KGaA**
**40589 Düsseldorf (DE)**

(72) Erfinder:
- **KNÜBEL, Georg**
**40219 Düsseldorf (DE)**
- **KOENEN, Annika**
**41516 Grevenbroich (DE)**

(56) Entgegenhaltungen:
**EP-A1- 1 138 374**
**DE-A1- 102007 050 434**
**DE-T2- 60 132 192**
**JP-A- 2007 212 140**
**US-A1- 2003 065 636**
**US-A1- 2004 122 782**
**US-A1- 2006 195 300**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 390 992 B1

## Beschreibung

Die Erfindung betrifft ein Verfahren zum computergestützten Ermitteln von Eigenschaften von Haarfarben nach Anspruch 1 und eine Datenverarbeitungsvorrichtung zum Ausführen des Verfahrens nach Anspruch 8.

Eigenschaften von Haarfarben können beispielsweise eine Haarfarbinformation, eine Waschechtheit, eine Lichtechtheit, eine Grauabdeckung oder weitere Eigenschaften aufweisen. Dabei können die Haarfarben mittels Färbens von Haar mit einem Haarfärbemittel, auch als Färbevorgang bezeichnet, erzeugt sein.

Haarfärbemittel können eine Mischung aus verschiedenen Farbstoffvorstufen aufweisen und können deshalb auch als Färbemischung bezeichnet werden.

Eine Vorhersage der genannten Eigenschaften von Haarfarben, also ein Ermitteln eines zu erwartenden Färbeergebnisses, ohne den Färbevorgang tatsächlich ausgeführt zu haben, kann vielfach kommerziell genutzt werden, beispielsweise bei einer Berechnung eines Farbresultates auf einer individuellen Ausgangshaarfarbe, bei einer Berechnung der optimalen Farben, z.B. von Parametern einer in einem Farbraum parametrisierten Haarfarbe, für eine Darstellung, z.B. auf Verpackungen, in Anzeigen (beispielsweise Werbeanzeigen), im Internet und in Apps, bei der Zusammenstellung eines individuellen ("customized") Haarfärbemittels und für eine nachfolgende Rezepturoptimierung in der Produktentwicklung, beispielsweise hinsichtlich der Waschechtheit, der Lichtechtheit und/oder der Grauabdeckung einer Haarfarbe.

Während es in verwandten Gebieten der Farberzeugung möglich ist, die wiederzugebende Farbe exakt zu berechnen, beispielsweise beim präzisen Fotodruck mittels kalibrierter Pigmentdrucker, ist dies im Bereich der Haarfarben bislang nicht möglich gewesen.

Ein Hauptgrund dafür ist, dass beim Haarefärben, also zum Erzeugen einer Haarfarbe, oft keine Farbstoffe eingesetzt werden, zumindest nicht unmittelbar, sondern Farbstoffvorstufen. Während eines Färbevorgangs kann sich dann eine Vielzahl von verschiedenen Farbstoffen bilden, deren colorimetrische Eigenschaften als Reinsubstanzen möglicherweise nicht komplett bekannt sind. Ferner können Konzentrationen der Farbstoffe in den gefärbten Haaren unbekannt sein, und es kann auch unbekannt sein, welche Konzentration der Farbstoffe in den gefärbten Haaren welcher Konzentration von Farbstoffvorstufen im Haarfärbemittel entspricht. Dies kann zumindest teilweise daran liegen, dass die Farbstoffvorstufen bei der Bildung der verschiedenen Farbstoffe miteinander wechselwirken.

Daher war bisher eine Berechnung von Reflexionsspektren von gefärbten Haaren nicht möglich.

Bekannte Verfahren zur Vorhersage, z.B. Berechnung, einer Farbe bedienen sich eines Prinzips von Vergleichsausfärbungen und Übertragung von so gewonnenen Resultaten auf Haare mit ähnlicher Ausgangshaarfarbe. Siehe hierzu beispielsweise die FR 2984569 B1, die JP 2007-212140 A, die JP 2004-144569 A oder die WO 2001/87245 A2.

Diese Verfahren sind allerdings zur Berechnung einer Farbe, oder auch anderer Eigenschaften, welche aus einer Färbung mit einer unbekannten, noch nie ausgefärbten Färbemischung resultieren würde, nicht geeignet.

DE 2007 050434 A1 beschreibt ein Verfahren und Anordnung zum computergestützten Ermitteln mindestens einer Eigenschaft einer Haarcoloration basierend auf einer Formulierung aus chemisch reaktiven und/oder unreaktiven Rohstoffen.

In verschiedenen Ausführungsbeispielen wird ein Verfahren zum computergestützten Ermitteln von Eigenschaften von Haarfarben bereitgestellt, wobei das Ermitteln auch für Haarfarben möglich sein kann, welche mit einer Färbemischung erzielt werden, welche noch nicht zuvor ausgefärbt wurde (bzw. deren Eigenschaften nicht schon vorher bekannt sind).

In verschiedenen Ausführungsbeispielen werden Methoden aus dem Feld der prädiktiven Analytik, für welche üblicherweise der entsprechende englische Begriff "Predictive Analytics" verwendet wird (auch bekannt als "Big Data" (sinngemäß übersetzt "große Datenmenge"), "Data Mining" (übersetzt "Auswertung großer Datenmengen") oder "Machine Learning" (übersetzt "Maschinenlernen")), genutzt, um trotz möglicherweise vieler Unbekannter in einer Färbemischung präzise Berechnungen von Eigenschaften, beispielsweise den oben genannten Eigenschaften, von Haarfarben zu ermöglichen.

Es ist gemäß verschiedenen Ausführungsbeispielen möglich, mittels Testfärbungen einen Datensatz (auch als Haarfarben-Daten bezeichnet) bereitzustellen, welcher für jede der Testfärbungen eine Mehrzahl von Färbevoraussetzungsparametern und mindestens einen Färbeergebnisparameter aufweist.

Die Mehrzahl von Färbevoraussetzungsparametern weist mindestens zwei Konzentrationen von Farbstoffvorstufen auf, wobei die Mehrzahl von Färbevoraussetzungsparametern ferner eine Grundhaarfarbe aufweist, wobei die Grundhaarfarbe in einem Farbraum parametrisiert ist, wobei die Mehrzahl von Färbevoraussetzungsparametern ferner eine Vorschädigung des Haars aufweist. Darüber hinaus kann der Datensatz jedoch weitere Färbevoraussetzungsparameter aufweisen, beispielsweise Konzentrationen weiterer Farbstoffvorstufen, weitere Inhaltsstoffe eines Haarfärbemittels, einen Ergrauungsgrad des Haars, und/oder andere Färbevoraussetzungsparameter.

Der mindestens eine Färbeergebnisparameter weist eine Haarfarbe auf, welche in einem Farbraum parametrisiert ist.

Unter einer "Farbe" kann hierin ein Zusammenwirken eines Farbtons (d.h. eines spektralen Farbeindrucks, auch als Buntton bezeichnet, was als das verstanden werden kann, was als die "eigentliche Farbe" angesehen wird), einer Farbintensität (d.h. wie intensiv die Farbe erscheint, z.B. verglichen mit einem neutralen Grau, was auch als Sättigung, Farbsättigung, Buntheit, Chromatizität, Chromazität oder Farbtiefe bezeichnet wird) und einer Helligkeit (d.h. wie hell oder dunkel die Farbe erscheint) verstanden werden.

In verschiedenen Ausführungsbeispielen kann die Farbinformation beispielsweise eine Parametrisierung in einem bekannten Farbraum aufweisen, beispielsweise in einem L*a*b*-Farbraum (wobei L* die Helligkeit einer Farbe angibt, a* den Grün- und Rotanteil und b* den Blau- und Gelbanteil der Farbe; mitunter wird hierin auch als verkürzende Schreibweise Lab bzw. einzeln L, a, bzw. b verwendet) in einem RGB-Farbraum durch Farbanteile in Rot, Grün und Blau, in einem CMYK-Farbraum durch Farbanteile in Cyan, Magenta, Gelb und Schwarz, oder in einem beliebigen anderen Farbraum.

Unter dem Begriff "Farbton" kann hierin, wie oben beschrieben, der spektrale Farbeindruck einer Farbe verstanden werden, unabhängig davon, wie dieser parametrisiert sein kann, beispielsweise als ein Punkt in einem zweidimensionalen Farbraum (z.B. a*b* des L*a*b*-Systems) oder ein Verhältnis von Farbanteilen (wie z.B. beim RGB-Farbraum oder beim CMYK-Farbraum).

In verschiedenen Ausführungsbeispielen kann ein Farbraum, dem die Farbinformation (z.B. die Haarfarbinformation des gefärbten Haars oder des Haars vor der Färbung, was auch als Grundhaarfarbe bezeichnet wird) entstammt, oder in welchem die Farbinformation dargestellt wird (beispielsweise wenn eine Haarfarbe dargestellt wird, siehe unten) so beschaffen sein, dass eine ermittelte oder dargestellte Farbe unabhängig von einem Medium ist, durch welches die Farbe ermittelt oder dargestellt wird (z.B. Farbmessgerät, Bildschirm, Drucker, Scanner, menschliches Auge, usw.). Der Farbraum kann beispielsweise ein L*a*b*-Farbraum sein, die Farbinformation ein beispielsweise mittels a* und b* parametrisierter Farbton. Die einheitliche Darstellung in dem mediumunabhängigen Farbraum kann es beispielsweise ermöglichen, ein realitätsnahes zu erwartendes Färbeergebnis zu präsentieren, beispielsweise indem eine mittels Färbens erzielte Farbe beim Betrachter des gefärbten Haars denselben Farbeindruck hinterlässt wie in einer Darstellung des zu erwartenden Ergebnisses, beispielsweise als ein Verpackungsaufdruck, eine Anzeige an einem Computerbildschirm, o.ä.

Der mindestens eine Färbeergebnisparameter kann in verschiedenen Ausführungsbeispielen ferner weitere Eigenschaften der gefärbten Haarfarbe aufweisen, beispielsweise eine Lichtechtheit, eine Waschechtheit oder eine Fähigkeit zur Grauabdeckung.

Der Datensatz wird als Grundlage für eine Anwendung eines Prädiktive-Analytik Verfahrens genutzt.

Beispielsweise können die Färbevoraussetzungsparameter oder ein Teil der Färbevoraussetzungsparameter und die ihnen zugeordneten Färbeergebnisparameter oder ein Teil der Färbeergebnisparameter genutzt werden, um ein Modell zu erzeugen, welches den Datensatz möglichst genau beschreibt.

In verschiedenen Ausführungsbeispielen kann es sich bei den Messdaten des Datensatzes, d.h. den gemessenen Angaben der Haarfarben-Daten, die Eigenschaften der mittels Färbens erzeugten Haarfarbe beschreiben (z.B. L*, a*, b* für die Farbe, eine Waschechtheit, Lichtechtheit, Grauabdeckung o.ä.), um abhängige Variablen handeln. Mittels eines komplexen mathematischen Modells, welches mittels des Prädiktive-Analytik-Verfahrens gefunden werden kann, kann die Abhängigkeit der abhängigen Variablen von den unabhängigen Variablen (beispielsweise den Konzentrationen der Farbstoffvorstufen, beispielsweise den Konzentrationen, wie sie auf dem Kopf ("on head") vorliegen) modelliert werden. Das heißt, mittels des Prädiktive-Analytik-Verfahrens kann eine Beziehung zwischen den unabhängigen und den abhängigen Variablen (anders ausgedrückt zwischen den Färbevoraussetzungsparametern und den Färbeergebnisparametern) ermittelt werden. Für die Farbe kann dies beispielsweise ausgedrückt werden als:

$$L^*a^*b^* = f(c_1, c_2, c_3, \ldots, c_n),$$

wobei L*a*b* für die Farbparameter steht, $c_i$ (i=1,...,n, n>1) für Konzentrationen von Farbstoffvorstufen. Dabei kann die Funktion analytisch bekannt sein oder auch nicht. Falls keine analytische Funktion bekannt ist, können die Werte der abhängigen Variablen (der Färbeergebnisparameter) auch mittels numerischer Algorithmen berechnet werden.

Neben Farbstoffvorstufen können auch Farbstoffe in Haarfärbemitteln eingesetzt werden. Entsprechend kann $c_i$ auch für $c_i$ für Konzentrationen von Farbstoffen stehen.

Mögliche unabhängige Variablen können metrisch (kardinal), ordinal oder kategorial sein.

In verschiedenen Ausführungsbeispielen können die unabhängigen Variablen (die Färbevoraussetzungsparameter) Eigenschaften sein, die das Färbeergebnis beeinflussen, beispielsweise die Konzentration einer jeweiligen der Farbstoffvorstufen, die Grundhaarfarbe, ein Schädigungszustand und/oder ein Ergrauungsgrad des Haars, oder Ähnliches.

In verschiedenen Ausführungsbeispielen kann mittels der Prädiktiven Analytik ein Modell erzeugt werden, welches bei vorgegebenen Färbevoraussetzungsparametern (unabhängigen Variablen, Beispiele siehe oben) die Färbeergebnisparameter (abhängige Variablen, Beispiele siehe oben) möglichst genau vorhersagt.

In verschiedenen Ausführungsbeispielen können mittels der Prädiktiven Analytik unabhängige Variablen identifiziert werden, welche keinen oder lediglich einen unbedeutenden Einfluss auf das Modell haben. Anders ausgedrückt kann es sein, dass unabhängige Variablen (Färbevoraussetzungsparameter) in den Haarfarben-Daten vorhanden sind, von welchen angenommen werden konnte, dass sie einen Einfluss auf die abhängigen Variablen (Färbeergebnisparameter) haben, obwohl dies nicht oder lediglich unbedeutend der Fall ist. Diese unbedeutenden Variablen können mittels des Prädiktive-Analytik-Verfahrens identifiziert werden und ggf. bei nachfolgenden Modellierungen mit vergleichbaren Voraussetzungen außer Betracht gelassen werden, um die Modellgüte zu steigern.

Mittels der Prädiktiven Analytik wird ein kontinuierliches Modell der Färbevoraussetzungsparameter und der Färbeergebnisparameter erzeugt, so dass es möglich ist, für einen Wert eines Färbevoraussetzungsparameters oder einer Kombination von Werten für eine Mehrzahl von Färbevoraussetzungsparametern, welche/r keinem der entsprechenden experimentellen Werte bzw. Wertekombinationen entspricht, mit Hilfe des Modells einen Wert für einen Färbeergebnisparameter zu ermitteln.

Es können auch kategoriale Eigenschaften, wie zum Beispiel "gut" oder "schlecht", modelliert werden.

Prädiktive Analytik kann allgemein als ein Verfahren beschrieben werden, um aus großen Datenmengen Informationen zu extrahieren und aus diesen Daten ein Modell zu erzeugen, welches es erlaubt, auch für Proben, die nicht Teil des Datensatzes sind, Vorhersagen zu treffen. Bei Anwendung eines Prädiktive Analytik Verfahrens kann typischerweise ein Teil des Datensatzes als Trainings-Datensatz (auch als Trainingssatz oder Trainingsdaten bezeichnet) genutzt werden. Anhand dieses Trainingsdatensatzes können ein oder mehrere Modelle erzeugt werden, welche dann anhand der Daten, die nicht Teil des Trainingsdatensatzes sind, anhand der gesamten Daten, oder anhand eines speziell ausgewählten Teils der Daten, getestet werden können.

Für eine Bewertung des Modells, also eine Ermittlung der Anpassungsgüte, können beispielsweise ein Bestimmtheitsmaß $R^2$, ein mittlerer absoluter Fehler, ein mittlerer quadratischer Fehler, eine Standardabweichung und/oder eine mittlere Abweichung herangezogen werden.

Das Bestimmtheitsmaß $R^2$ kann für ein lineares Regressionsmodell einem quadrierten Korrelationskoeffizienten entsprechen. Für ein anderes Modell (eine andere Beziehung) kann es anders definiert sein.

Für die Modellierung mittels Prädiktiver Analytik können gemäß verschiedenen Ausführungsbeispielen verschiedene Funktionen oder Verfahren herangezogen werden. In einem einfachen Fall kann beispielsweise eine multiple (lineare) Regression genutzt werden. Bessere Ergebnisse können typischerweise unter Verwendung von polynomen Regressionen, neuronalen Netzen, Support Vector Machines, Entscheidungsbäumen (beispielsweise Tree-Ensembles) oder ähnlichem erzielt werden.

In verschiedenen Ausführungsbeispielen kann das beschriebene Verfahren zur computergestützten Vorhersage von Eigenschaften von Haarfarben mittels einer Datenverarbeitungsvorrichtung ausgeführt werden.

Die Datenverarbeitungsvorrichtung kann beispielsweise einen Computer aufweisen, oder jede andere Datenverarbeitungsvorrichtung, die geeignet ist, die Daten zu speichern und bereitzustellen und das Prädiktive Analytik Verfahren auszuführen, also beispielsweise jede Datenverarbeitungsvorrichtung mit hinreichend großem Datenspeicher und hinreichend leistungsfähigem Prozessor.

In verschiedenen Ausführungsbeispielen kann die Datenverarbeitungsvorrichtung mindestens eine Eingabevorrichtung zum Eingeben von Informationen in die Datenverarbeitungsvorrichtung aufweisen, beispielsweise zum Eingeben der Haarfarben-Daten und ggf. zum Eingeben von Anweisungen, Parametern usw. für ein Ausführen des Verfahrens.

In verschiedenen Ausführungsbeispielen kann die Datenverarbeitungsvorrichtung mindestens eine Ausgabevorrichtung zum Ausgeben von Informationen aufweisen, beispielsweise zum Ausgeben von Ergebnissen des Verfahrens.

In verschiedenen Ausführungsbeispielen kann die mindestens eine Ausgabevorrichtung einen Bildschirm und/oder einen Drucker aufweisen.

In verschiedenen Ausführungsbeispielen, beispielsweise wenn die auszugebenden Färbeergebnisparameter eine Haarfarbe aufweisen, kann die Farbe auch für die Ausgabe in einem mediumunabhängigen Farbraum, z.B. dem L*a*b*-Farbraum, parametrisiert sein. Dadurch kann es ermöglicht werden, dass beispielsweise das wie oben beschrieben ermittelte zu erwartende Färbeergebnis, welches beispielsweise an einem Bildschirm angezeigt oder ausgedruckt werden kann (beispielsweise auf eine Verpackung eines Färbeprodukts), im Wesentlichen so erscheint, wie es nach einer Färbung auch in Wirklichkeit erscheinen würde. Sofern die Ausgabevorrichtung eine andere Parametrisierung der Farbe erfordert, kann die ermittelte Farbe von einem Farbraum in einen anderen transformiert werden.

Es wird ein Verfahren zum computergestützten Ermitteln von Eigenschaften von Haarfarben nach Anspruch 1 bereitgestellt. Das Verfahren weist unter anderem auf: Bereitstellen von Haarfarben-Daten, wobei die Haarfarben-Daten für eine Mehrzahl von Färbevorgängen jeweils Werte für eine Mehrzahl von Färbevoraussetzungsparametern und für mindestens einen Färbeergebnisparameter aufweisen, wobei für jeden Färbevorgang der Mehrzahl von Färbevorgängen die Mehrzahl von Färbevoraussetzungsparametern eine erste Konzentration einer ersten Farbstoffvorstufe und eine zweite Konzentration einer zweiten Farbstoffvorstufe aufweist, wobei für jeden Färbevorgang der Mehrzahl von Färbevorgängen der mindestens eine Färbeergebnisparameter eine gemessene Angabe über eine Eigenschaft von Haarfarben aufweist, und Ermitteln einer Beziehung zwischen der Mehrzahl von Färbevoraussetzungsparametern und dem mindestens einen Färbeergebnisparameter mittels prädiktiver Analytik basierend auf den Haarfarben-Daten.

Das Verfahren weist ferner auf: Bestimmen, mittels der ermittelten Beziehung, eines Werts für einen Färbeergebnisparameter zu einer Wertekombination aus jeweils einem Wert für eine ausgewählte Mehrzahl von Färbevoraussetzungsparametern, wobei die Wertekombination für keinen der Färbevorgänge mit dessen Wertekombination für die ausgewählte Mehrzahl von Färbevoraussetzungsparametern identisch ist.

In verschiedenen Ausführungsformen kann die prädiktive Analytik mindestens ein Verfahren nutzen aus einer Gruppe von Verfahren, wobei die Gruppe von Verfahren aufweisen kann: lineare oder multi-lineare Regression, polynome Regression, multiple polynome Regression, Neuronale-Netze-Verfahren, Support Vector Machine-Verfahren, und Entscheidungsbäume-Verfahren (inklusive Tree-Ensembles).

In verschiedenen Ausführungsformen kann das Entscheidungsbäume-Verfahren Entscheidungsbaum-Ensembles nutzen.

Vorteile von Entscheidungsbaum-Ensembles in diesem Verfahren ist, dass sie genauere Vorhersagen liefern und dass sie einen geringeren Rechenaufwand als andere Verfahren der prädiktiven Analytik benötigen. Dies kann vorteilhaft sein, wenn mehrere Verfahren zum computergestützten Ermitteln von Eigenschaften von Haarfarben parallel auf einem Server durchgeführt werden sollen.

Die Mehrzahl von Färbevoraussetzungsparametern weist ferner eine Grundhaarfarbe auf, wobei die Grundhaarfarbe in einem Farbraum parametrisiert ist.

Die Mehrzahl von Färbevoraussetzungsparametern weist ferner eine Vorschädigung des Haars auf.

Die Vorschädigung kann durch physikalische Größen, wie beispielsweise das E-Modul, oder chemische Größen, wie zum Beispiel den Cysteinsäure-Gehalt, charakterisiert werden.

In verschiedenen Ausführungsformen kann die Mehrzahl von Färbevoraussetzungsparametern ferner einen Ergrauungsgrad aufweisen.

In verschiedenen Ausführungsformen kann der mindestens eine Färbeergebnisparameter mindestens eine Eigenschaft von Haarfarben aufweisen, wobei die Eigenschaften von Haarfarben aufweisen können: einen oder mehr Parameter einer in einem Farbraum parametrisierten Haarfarbe, eine Waschechtheit, eine Lichtechtheit, und eine Fähigkeit zur Grauabdeckung.

In verschiedenen Ausführungsformen kann der mindestens eine Färbeergebnisparameter eine Mehrzahl von Färbeergebnisparametern aufweisen.

Es wird eine Datenverarbeitungsvorrichtung nach Anspruch 8 bereitgestellt zum Ausführen eines computergestützten Ermittelns von Eigenschaften von Haarfarben, wobei die Datenverarbeitungsvorrichtung eingerichtet ist, das Verfahren gemäß Anspruch 1 auszuführen.

Die Datenverarbeitungsvorrichtung weist einen Prozessor auf, wobei der Prozessor eingerichtet sein kann, das Ermitteln der Beziehung auszuführen.

Ausführungsbeispiele der Erfindung sind in den Figuren dargestellt und werden im Folgenden näher erläutert.

Es zeigen

| | |
|---|---|
| Figur 1 | eine schematische Darstellung eines Prädiktive-Analytik-Verfahrens gemäß dem CRISP-Modell; |
| Figur 2 | ein Ablaufschema, welches funktionelle Komponenten und ihr Zusammenwirken bei einer Ausführung eines Verfahrens zum computergestützten Ermitteln von Eigenschaften von Haarfarben gemäß verschiedenen Ausführungsbeispielen darstellt; |
| Figur 3 | ein Diagramm, welches ein Ergebnis eines Verfahrens zum computergestützten Ermitteln von Eigenschaften von Haarfarben gemäß verschiedenen Ausführungsbeispielen darstellt; |
| Figur 4 | ein Ablaufschema, welches funktionelle Komponenten und ihr Zusammenwirken bei einer Ausführung eines Verfahrens zum computergestützten Ermitteln von Eigenschaften von Haarfarben gemäß verschiedenen Ausführungsbeispielen darstellt; |
| Figur 5A und 5B | zwei Diagramme, welche auf unterschiedliche Weise ein Ergebnis eines Verfahrens |

zum computergestützten Ermitteln von Eigenschaften von Haarfarben gemäß einem Ausführungsbeispiel darstellen;

| | |
|---|---|
| Figur 6A | und 6B zwei Diagramme, welche auf unterschiedliche Weise ein Ergebnis eines Verfahrens zum computergestützten Ermitteln von Eigenschaften von Haarfarben gemäß einem Ausführungsbeispiel darstellen; |
| Figur 7 | ein Diagramm, welches ein Ergebnis eines Verfahrens zum computergestützten Ermitteln von Eigenschaften von Haarfarben gemäß einem Ausführungsbeispiel darstellt; |
| Figur 8 | ein Diagramm, welches ein Ergebnis eines Verfahrens zum computergestützten Ermitteln von Eigenschaften von Haarfarben gemäß einem Ausführungsbeispiel darstellt; |
| Figur 9 | ein Diagramm, welches ein Ergebnis eines Verfahrens zum computergestützten Ermitteln von Eigenschaften von Haarfarben gemäß einem Ausführungsbeispiel darstellt; |
| Figuren 10A, 10B und 10C | drei Diagramme, welche das Verhältnis von Messpunkten, wahrer Funktion und Modell für verschiedene bei einem Verfahren zum computergestützten Ermitteln von Eigenschaften von Haarfarben gemäß verschiedenen Ausführungsbeispielen verwendeten Modellen darstellen; |
| Figur 11 | ein Ablaufdiagramm, welches ein Verfahren zum computergestützten Ermitteln von Eigenschaften von Haarfarben gemäß verschiedenen Ausführungsbeispielen darstellt; |
| Figur 12 | eine schematische Darstellung einer Datenverarbeitungsvorrichtung gemäß verschiedenen Ausführungsbeispielen; und |
| Figur 13 | Haarfarben-Daten gemäß einem Ausführungsbeispiel. |

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

Figur 1 zeigt eine schematische Darstellung 100 eines Prädiktive-Analytik-Verfahrens.

Hierin werden die Begriffe Prädiktive Analytik, Predictive Analytics, Big Data und Data Mining synonym verwendet.

Als Ausgangspunkt eines jeden Prädiktive-Analytik-Verfahrens kann, wie in Fig. 1 dargestellt ist, das Verstehen des Geschäfts, beispielsweise dessen, was mit dem Prädiktive-Analytik-Verfahren erreicht werden soll, gesehen werden.

Dem Prädiktive-Analytik-Verfahren benötigt Daten, um ausgeführt werden zu können. Diese können beispielsweise bereits vorliegen oder zielgerichtet zusammengetragen werden. Ein Verständnis dessen, welche Daten für das gesetzte Ziel hilfreich sein können und welche Information in Form dieser Daten vorliegt, kann zweckdienlich sein, beispielsweise vor einem Vorbereiten der Daten für die Verwertung durch das Prädiktive-Analytik-Verfahren, also beispielsweise bevor die Daten in ein Computerprogramm zur Durchführung des Verfahrens eingelesen werden.

Anschließend kann eine Modellierung mittels des Prädiktive-Analytik-Verfahrens durchgeführt werden. Eine anschließende Evaluierung des Ergebnisses kann beispielsweise zu einer unmittelbaren Verwendung des Ergebnisses führen, oder beispielsweise dazu, dass das Verständnis des Geschäfts beeinflusst wird, beispielsweise indem erkannt wird, dass weitere Parameter einbezogen werden sollten, oder die gestellte Aufgabe so nicht lösbar ist.

In Fig.1 ist mittels des Rahmens 110 angedeutet, auf welche Teile eines solchen Prädiktive-Analytik-Verfahrens das Verfahren zum computergestützten Ermitteln von Eigenschaften von Haarfarben gemäß verschiedenen Ausführungsbeispielen bei Vorliegen des Datensatzes beispielsweise Einfluss nehmen kann, nämlich auf die Modellierung und die Evaluierung.

Tabelle 1 und Tabelle 2 stellen zwei beispielhafte Haarfarben-Daten (bzw. Auszüge aus einem umfangreicheren Datensatz von Haarfarben-Daten) dar.

Zum Erzeugen von gemessenen Angaben für die Haarfarben wurden die jeweiligen Haarfärbemittel (das in Spalte 1 jeweils aufgelistete Produkt, 74 verschiedene Produkte (Rezepturen) bei Tabelle 1 und 53 verschiedene Produkte (Rezepturen) bei Tabelle 2) gemäß Gebrauchsanweisung ausgefärbt (auf Kerling Euronaturhaar weiß) und farbmetrisch vermessen (Lichtart D65/10, diffus mit Glanz, 8°).

In Tabelle 1 ist in Spalte 2 das unmittelbare Ergebnis der farbmetrischen Vermessung als Parameter im L*a*b*-Farbraum angegeben.

In Tabelle 2 wurde die farbmetrische Vermessung zweimal durchgeführt, einmal unmittelbar nach dem Ausfärben und einmal nach 12 Haarwäschen. Der daraus ermittelte Farbabstand $\Delta E_{00}$ ist in Spalte 2 der Tabelle 2 notiert. Der Farbabstand kann als Maß für eine Waschechtheit der Haarfarbe genutzt werden.

In den Spalten 3 und 4 der Tabellen 1 und 2 sind jeweils Konzentrationen zweier Farbstoffvorstufen (p-Toluylendiaminsulfat und m-Aminophenol) aufgelistet, wie sie auf dem Kopf vorliegen würden. Insgesamt wurden Konzentrationen für zwanzig Farbstoffvorstufen ermittelt, von denen hier lediglich zwei beispielhaft wiedergegeben sind.

Tabelle 2 ist ferner in Fig.13 abgebildet. Die beispielhaften Haarfarben-Daten 1300 weisen für eine Mehrzahl von Färbevorgängen (davon sind zwei beispielhaft mit 1310 gekennzeichnet) eine Mehrzahl von Färbevoraussetzungsparametern 1330 (die Konzentrationen der beiden Farbstoffvorstufen) und einen Färbeergebnisparameter 1320 (den Farbabstand nach 12 Haarwäschen) auf. Ein beispielhafter Wert für einen der Färbevoraussetzungsparameter ist mit 1331 gekennzeichnet. Ein beispielhafter Wert für den Färbeergebnisparameter ist mit 1321 gekennzeichnet.

Die in den Tabellen wiedergegebenen Daten sind lediglich als veranschaulichende Beispiele zu verstehen. Welche Haarfarben-Messdaten in verschiedenen Ausführungsbeispielen erzeugt und für das Verfahren genutzt werden, hängt von einer konkreten Anwendung ab. Beispielsweise können andere, weniger oder mehr Produkte verwendet werden, welche ggf. andere, mehr oder weniger Farbstoffvorstufen aufweisen können, anstelle von Farbe oder Farbabstand können andere Parameter bestimmt und in den Haarfarben-Daten angegeben werden, wie beispielsweise eine Lichtechtheit, eine Grundhaarfarbe, usw.

Ein Bereitstellen der Haarfarben-Daten als Tabelle ist lediglich beispielhaft. Die Haarfarben-Daten können in jeder beliebigen Form bereitgestellt werden, welche ein Zuordnen der Färbeergebnisparameter zu den jeweiligen zugehörigen Färbevoraussetzungsparametern und ein computergestütztes Verwenden der Haarfarben-Daten ermöglicht.

Tabelle 1

| Produkt | L a b | p-Toluylen-diaminsulfat [μmol/ 100g] | m-Aminophenol [μmol / 100g] |
|---|---|---|---|
| N&E 542 Mittelaschblond | 40,4 3,8 11,5 | 859,5 | 130,6 |
| N&E 545 Mittelgoldblond | 38,7 7,3 12,7 | 1369,6 | 75.5 |
| N&E 550 Dunkelblond | 42,7 4 0 15,2 | 862,4 | 66.4 |
| N&E 555 Dunkles Goldblond | 38,5 7,6 13,0 | 089.8 | 72,9 |
| N&E 557 Multi-Reflex-Braun | 31,7 8,9 13.7 | 1859,6 | 0,0 |
| N&E 550 Hellbraun | 26,5 3,5 7,6 | 2117,1 | 400.9 |
| N&E 562 Hellaschbraun | 29,5 3,2 7,4 | 1505,7 | 184.5 |
| M&E 555 Hellgoldbraun | 33,4 5,5 11,4 | 1811,S | 166,3 |
| N&E 566 Cinnamon Golden Brown | 25,2 6,7 10,0 | 3453,3 | 234,0 |
| N&E 568 Intensiv-Rot | 31,9 30,8 20,6 | 0,0 | 2000,1 |
| N&5 570 Mittelbraun | 27,0 3,7 9,6 | 2850,1 | 422,7 |
| N&E 574 Zartbitterschokolade | 25,9 3,0 10,1 | 3861,5 | 0,0 |
| N&E 575 Kastanie Rotbraun | 25.5 8,3 7,7 | 2810,3 | 304,3 |
| N&E 580 Dunkelbraun | 20,8 2,1 4,5 | 3758,4 | 989,7 |
| N&E 584 Mokkaschokolade | 23,7 6 5 7,3 | 3595,9 | 334,8 |
| N&E 585 Multi-Reflex-Braun | 23,3 8,3 5,2 | 2923,5 | 229,1 |
| N&E 586 Cinnamon Dark Brown | 23,2 7,1 8,7 | 5616,1 | 0,0 |
| N&E 588 Glossy Acaiberry | 22,9 15,5 2.9 | 1999,3 | 0,0 |
| N&E 590 Schwarz | 15,3 0,1 -1.7 | 8475,1 | 918,4 |

(fortgesetzt)

| Produkt | L a b | p-Toluylen-diaminsulfat [µmol/ 100g] | m-Aminophenol [µmol / 100g] |
|---|---|---|---|
| Nectra 688 | 25,6 10 4 6,6 | 0,0 | 2776,8 |
| Nectra 499 | 20,7 11,6 -2,0 | 447,2 | 336,7 |
| Nectra 568 | 29,6 10,4 6,6 | 1804,3 | 401,7 |
| Nectra 400 | 21.9 1,82,7 | 4133,8 | 1093,5 |
| Syoss Oleo 3-10 | 20,3 2,3 2.7 | 3759,4 | 938,7 |
| Syoss Oleo 5-92 | 30,5 31,7 13,2 | 0,0 | 1963,3 |
| Nectra 320 | 19,2 1,3 1.9 | 5650,4 | 1484,6 |
| Nectra 468 | 29,2 11,5 10,5 | 2338,3 | 0,0 |
| Farbcreme 1 | 19,3 6,4 -2,9 | 1001,1 | 0,0 |
| Farbcreme 2 | 20,4 7,3 -4.6 | 998,& | 0,0 |
| Farbcreme 3 | 19,6 5.8 -7,1 | 998.9 | 0,0 |
| Farbcreme 4 | 22,0 15,7 2.6 | 497,2 | 0,0 |
| Farbcreme 5 | 20,3 9,4 -3,5 | 501,7 | 0,0 |
| Farbcreme 6 | 22,6 10,5 -5,1 | 499,4 | 0,0 |
| Farbcreme 7 | 20,4 3,3 -4,5 | 751.4 | 0,0 |
| Farbcreme 8 | 23,3 10,1 -4,8 | 499,4 | 0,0 |
| Farbcreme 9 | 22.5 3,3 -9,3 | 499,4 | 0,0 |
| Farbcreme 10 | 27,8 19,8 2,6 | 249,7 | 0,0 |
| Farbcreme 11 | 24,5 13,5 -5,1 | 252,0 | 0,0 |
| Farbcreme 12 | 30,1 13,7 -5,8 | 249,7 | 0,0 |
| Farbcreme 13 | 25,2 12,5 -4,8 | 376.3 | 0,0 |
| Farbcreme 14 | 25,2 12,5 -5,2 | 333,7 | 0,0 |
| Farbcreme 15 | 29.1 12.7 -5.0 | 333,7 | 0,0 |
| Farbcreme 16 | 27,9 9,1 -9,7 | 333,7 | 0,0 |
| Farbcreme 17 | 25.1 20,7 2.6 | 165,7 | 0,0 |
| Farbcreme 18 | 26,0 15,1 -5.5 | 168,0 | 0,0 |
| Farbcreme 19 | 35,1 14,4 -4,1 | 165.7 | 0,0 |
| Kaschmirrot Variante 2 | 31,4 36,1 22,6 | O.C | 0,0 |
| Syoss Color 2014 5-22 | 25,3 25,3 12,5 | 491,7 | 2812,0 |
| Syoss Color 2014 1-4 | 15.0 12 -3,3 | 4994,3 | 0.0 |
| Syoss Color 2014 5-28 | 23,2 9,1 9,6 | 1756,7 | 166,0 |
| Syoss Oleo 4-29 | 25,7 26,0 13,7 | 681,3 | 227,7 |
| Syoss Oleo 1-40 | 15,3 1.4 -3,3 | 4994,3 | 0,0 |
| Igora Royal 5-88 | 25,1 26,5 12,6 | 481.7 | 2812,0 |
| Nectra 600 | 29,8 4,6 10,0 | 1770,7 | 252,0 |
| Nectra 662 | 29,3 6,7 9,4 | 2179,3 | 228,1 |
| Nectra 777 | 45,3 29,7 33,2 | 0,0 | 620,0 |
| Igora Royal 3-0 | 18,3 1,1 1,8 | 5788.6 | 1512,1 |
| Igora Royal 5-5 | 31,0 6,7 11,3 | 2043,1 | 165,0 |

(fortgesetzt)

| Produkt | L a b | p-Toluylen-diaminsulfat [μmol/ 100g] | m-Aminophenol [μmol / 100g] |
|---|---|---|---|
| Igora Royal 6-65 | 33,0 7,1 11,8 | 1793,4 | 183,3 |
| Igora Royal 6-0 | 28,84,1 8,5 | 1922.6 | 440,3 |
| Igora Royal 5-1 | 28.3 1,4 5,2 | 1566,4 | 247,4 |
| Igora Royal 4-88 | 25.1 26,5 14,4 | 794.6 | 1374,6 |
| Igora Royal 7-887 | 35,1 40,7 29,0 | 149.5 | 0,0 |
| Syoss Color 2012 4-2 | 25.5 242 13.3 | 1589,1 | 458,2 |
| Syoss Color 2012 5-22 | 3D,3 31.6 19,3 | 612.9 | 481,1 |
| Syoss Color 2012 1-4 | 16,8 1,7 -5,4 | 4904,3 | 0.0 |
| Syoss Color 2D12 5-29 | 33,5 36,1 22.7 | 0,0 | 2000,1 |
| Syoss Color 2012 3-55 | 27,7 6,9 9,3 | 3904,7 | 412,4 |
| Syoss Color 2012 5-0 | 25,6 3.0 6.6 | 3132.8 | 710,2 |
| Brilliance 880 | 25,2 25 6.3 | 3408.6 | 740,9 |
| Igora Royal 1-0 | 16,8 0,1 -1.6 | 7355.3 | 733,1 |
| Syoss Oleo 2-10 | 15.7 1,1 0,3 | 5012,5 | 1319,6 |
| Syoss Oleo 4-18 | 24,9 6,3 8,2 | 2042,8 | 131,5 |
| Syoss Oleo 6-10 | 27,4 46 10,6 | 2144,4 | 163,1 |

Tabelle 2

| Produkt | $dE_{00}$ (12HW) | p-Toluylen-diaminsulfat [μmol/ 100g] | m-Aminophenol [μmol / 100g] |
|---|---|---|---|
| N&E 542 Mittelaschblond | 2,7 | 859,5 | 130,6 |
| N&E 545 Mittelgoldblond | 1,4 | 1369,6 | 75,6 |
| N&E 550 Dunkelblond | 2,4 | 862,4 | 56,4 |
| N&E 555 Dunkles Goldblond | 2,7 | 989.6 | 72,9 |
| N&E 557 Multi-Reflex-Braun | 1,8 | 1959,6 | 0,0 |
| N&E 560 Hellbraun | 2,8 | 2117,1 | 400,9 |
| N&E 562 Heilaschbraun | 2.9 | 1505,7 | 184,5 |
| N&E 565 Hellgoldbraun | 2,0 | 1811,9 | 166,3 |
| N&E 566 Cinnamon Golden Brown | 1,6 | 3453,3 | 234,0 |
| N&E 568 Intensiv-Rot | 3,4 | 0,0 | 2000,1 |
| N&E 570 Mittelbraun | 1,8 | 2650,1 | 422,7 |
| N&E 574 Zartbitterschokolade | 1,6 | 3861,9 | 0,0 |
| N&E 576 Kastanie Rotbraun | 1,4 | 2810,3 | 304,3 |
| N&E 580 Dunkelbraun | 2,3 | 3759,4 | 989,7 |
| N&E 584 Mokkaschokolade | 1,4 | 3595,9 | 384,9 |
| N&E 585 Multi-Reflex-Braun | 1,9 | 2928,5 | 229,1 |
| N&E 586 Cinnamon Dank Brown | 1,3 | 5616,1 | 0,0 |
| N&E 588 Glossy Acaiberry | 3,1 | 1999,3 | 0,0 |
| N&E 590 Schwarz | 0,5 | 6475,1 | 916,4 |

(fortgesetzt)

| Produkt | $dE_{00}$ (12HW) | p-Toluylen-diaminsulfat [µmol/ 100g] | m-Aminophenol [µmol / 100g] |
|---|---|---|---|
| Nectra 688 | 2,2 | 0.0 | 2776,8 |
| Nectra 499 | 1,9 | 4472 | 386,7 |
| Nectra 568 | 1,9 | 1804,8 | 491,7 |
| Nectra 400 | 1,8 | 4133,9 | 1090,5 |
| Syoss Oleo 3-10 | 2,3 | 3759,4 | 989,7 |
| Syoss Oleo 5-92 | 2,8 | 0,0 | 1963,9 |
| Nectra 300 | 1,1 | 5650,4 | 1484,6 |
| Nectra 468 | 1,4 | 2338.3 | 0,0 |
| Kaschmirrot Variante 2 | 2,8 | 0,0 | 0,0 |
| Syoss Color 2014 5-22 | 2,8 | 491,7 | 2812,0 |
| Syoss Color 2014 1-4 | 2,1 | 4994,3 | 0,0 |
| Syoss Color 2014 5-28 | 1,7 | 1736,7 | 165,0 |
| Syoss Oleo 4-29 | 4,4 | 681,3 | 227,7 |
| Syoss Oleo 1-40 | 2,5 | 4994,3 | 0,0 |
| Igora Royal 5-88 | 2,8 | 491,7 | 2812,0 |
| Nectra 777 | 7,4 | 0,0 | 630,0 |
| Igora Royal 3-0 | 2,2 | 5768,9 | 1512,1 |
| Igora Royal 5-5 | 1,7 | 2043,1 | 165,0 |
| Igora Royal 6-65 | 1,8 | 1793,4 | 183,3 |
| Igora Royal 6-0 | 2,9 | 1922,6 | 440,3 |
| Igora Royal 6-1 | 3,7 | 1566,4 | 247,4 |
| Igora Royal 4-88 | 2,6 | 794,6 | 1374,6 |
| Igora Royal 7-887 | 2,2 | 149,8 | 0,0 |
| Syoss Color 2012 4-2 | 4,1 | 1589,1 | 458,2 |
| Syoss Color 2012 5-22 | 2,8 | 612,9 | 481,1 |
| Syoss Color 2012 1-4 | 2,4 | 4994,3 | 0,0 |
| Syoss Color 2012 5-29 | 3,2 | 0,0 | 2000,1 |
| Syoss Color 2012 3-65 | 1,3 | 3904,7 | 412,4 |
| Syoss Cofor 2012 5-0 | 1,9 | 3132,8 | 710,2 |
| Brillance 880 | 1,5 | 3408,6 | 740,9 |
| Igora Royal 1-0 | 1,0 | 7355,3 | 733,1 |
| Syoss Oleo 2-10 | 1,8 | 5012,5 | 1319,6 |
| Syoss Oleo 4-18 | 2,6 | 2042,9 | 181,5 |
| Syoss Oleo 6-10 | 2,4 | 2144,4 | 163,1 |

Im vorliegenden Beispiel aus Tabelle 1 können die Konzentrationen der Farbstoffvorstufen (aus Spalten 3 und 4 der Tabelle 1) zwei Färbevoraussetzungsparameter, also unabhängige Variablen für das Prädiktive-Analytik-Verfahren, darstellen (eigentlich wären es mehr Färbevoraussetzungsparameter, nämlich ungefähr 100, entsprechend den ungefähr 100 Farbstoffvorstufen). Die Haarfarben-Daten können für jeden Färbevorgang für jeden Färbevoraussetzungsparameter einen Wert aufweisen, im vorliegenden Beispiel also 74 Werte (entsprechend den 74 Testfärbungen) für jede Farbstoffvorstufenkonzentration.

Die L*a*b*-Werte aus Spalte 2 der Tabelle 1 können beispielsweise Färbeergebnisparameter bilden, z.B. drei Färbeergebnisparameter L*, a* und b*, oder einen (dreidimensionalen) Färbeergebnisparameter. Die Färbeergebnisparameter können abhängige Variablen für das Prädiktive-Analytik-Verfahren darstellen. Für den/die Färbeergebnisparameter kann/können (ein) Wert/e angegeben sein, beispielsweise 74 Werte entsprechend den 74 Testfärbungen (auch als Färbevorgänge bezeichnet) für jeden Färbeergebnisparameter. Jeder Wert eines Färbeergebnisparameters entspricht dabei einem Zeileneintrag der dem Färbeergebnisparameter entsprechenden Spalte. Ein Wert eines Färbevoraussetzungsparameters kann auch Null sein. Ein Wert eines Färbeergebnisparameters kann auch Null sein.

Das in Tabelle 2 wiedergegebene Beispiel ist ähnlich dem in Tabelle 1, allerdings ist hier in Spalte 2 anstelle einer Farbe ein Farbabstand $\Delta E_{00}$ (der auch mit $dE_{00}$ bezeichnet wird) angegeben. Dieser bezeichnet hier den Abstand im Lab-Farbraum, den eine Haarfarbe nach einer vorgegebenen Anzahl von Haarwäschen erreicht (hier beispielsweise 12, üblich sind jedoch auch 24 oder 36 Wäschen, möglich wäre jedoch auch jede andere Zahl von Haarwäschen, die ein aussagekräftiges Ergebnis ermöglicht). Der Farbabstand kann als Maß für eine Waschechtheit (auch als Waschbeständigkeit bezeichnet) der Haarfarbe dienen. Je kleiner der erreichte Farbabstand ist, desto waschbeständiger ist die Farbe.

Ausgehend von bereitgestellten Haarfarben-Daten (beispielsweise wie in Tabelle 1 auszugsweise dargestellt) kann mittels prädiktiver Analytik eine Beziehung zwischen den Färbevoraussetzungsparametern (im vorliegenden Beispiel 14, von denen zwei in der Tabelle 1 bzw. der Tabelle 2 wiedergegeben sind) und dem gewünschten Färbeergebnisparameter (beispielhaft wird hier L* verwendet, die Helligkeit der Farbe, auch als Luminanz bezeichnet) ermittelt werden.

Alternativ wäre es in verschiedenen Ausführungsbeispielen möglich, andere Färbevoraussetzungsparameter zu nutzen, beispielsweise nur einen Teil der Farbstoffvorstufen-Konzentrationen heranzuziehen, weitere hinzuzufügen, weitere Parameter beispielweise durch Messungen zu ermitteln und den Haarfarben-Daten hinzuzufügen (z.B. Grundhaarfarbe, Ergrauungsgrad, Vorschädigung des Haars, o.ä.), und/oder andere Färbeergebnisparameter zu nutzen, beispielsweise die Haarfarbe (als dreidimensionalen Färbeergebnisparameter), die Waschechtheit (siehe das Beispiel aus Tabelle 2), o.ä., und für das Ermitteln der Beziehung heranzuziehen.

In verschiedenen Ausführungsbeispielen kann für eine Modellierung mittels prädiktiver Analytik jedes beliebige Programm genutzt werden, welches eine solche Funktionalität bereitstellt.

In den im Folgenden beschriebenen Beispielen wurde die prädiktive Analytik mittels der Software KNIME 2.11.2 durchgeführt.

Figur 2 zeigt ein Ablaufschema 200, welches funktionelle Komponenten (so genannte Knoten) und ihr Zusammenwirken bei einer Ausführung eines Verfahrens zum computergestützten Ermitteln von Eigenschaften von Haarfarben gemäß verschiedenen Ausführungsbeispielen darstellt.

Die funktionellen Komponenten (Knoten) können einzelne während der Ausführung des Prädiktive-Analytik-Verfahrens durchgeführte Prozesse darstellen.

Beispielsweise stellt Knoten 1 einen so genannten XLS Leser 210 dar, welcher gemäß verschiedenen Ausführungsbeispielen ein Einlesen von Haarfarben-Daten ausführen kann, wobei die Haarfarben-Daten beispielsweise in einem Excel-Format vorliegen können oder mittels des XLS-Lesers 210 in ein solches eingelesen werden können. So könnte beispielsweise die Tabelle 1 in vier Spalten mit jeweils 74 Zeilen eingelesen werden (sofern die drei Farbwerte L*, a* und b* in eine gemeinsame Spalte eingelesen werden), oder beispielsweise in 6 Spalten mit jeweils 74 Zeilen (falls drei Farbparameter L*, a* und b* jeweils in eine eigene Spalte eingelesen werden). Für die weitere Beschreibung des Beispiels sei angenommen, dass jeder der Farbparameter L*, a* und b* in eine eigene Spalte eingelesen worden ist.

In Knoten 2 kann gemäß verschiedenen Ausführungsbeispielen ein Spaltenfilter 220 bereitgestellt sein, welcher für eine Auswahl von als Färbevoraussetzungsparametern und von als Färbeergebnisparameter/n zu verwendenden Spalten genutzt werden kann. Ein Auswählen der Spalten kann nach dem Einlesen der Haarfarben-Daten erfolgen. Im Beispiel aus Tabelle 1 können die Spalten mit Konzentrationen von Farbstoff-Vorstufen als Färbevoraussetzungsparameter (unabhängige Variablen) ausgewählt werden, die Spalte mit der Helligkeit L* als Färbeergebnisparameter. Die ausgewählten Daten können auch als Trainingsset bezeichnet werden.

In Knoten 10 kann gemäß verschiedenen Ausführungsbeispielen eine funktionelle Komponente 230 zum Ausführen einer einfachen linearen Regression bereitgestellt sein. Die einfache lineare Regression kann nach dem Auswählen der für die Regression zu verwendenden Spalten ausgeführt werden. Mittels der Regression kann eine Beziehung zwischen der Mehrzahl von Färbevoraussetzungsparametern und dem mindestens einen Färbeergebnisparameter ermittelt werden. Diese gefundene Beziehung kann auch als Modell bezeichnet werden. Ein Prinzip für eine Anpassung des Modells an die Daten kann eine Optimierung des kleinsten-Quadrate-Fehlers sein. Anders ausgedrückt kann ein Fehler mittels der Methode der kleinsten Quadrate minimiert werden.

In verschiedenen Ausführungsbeispielen kann in Knoten 11 ein so genannter Weka Vorhersager 240 bereitgestellt sein, welchem sowohl die Ergebnisse der einfachen linearen Regression aus Knoten 10 als auch die ungenutzten Haarfarben-Daten aus Knoten 2 zugeführt werden können. Der Weka-Vorhersager 240 kann für sämtliche ausgewählten Färbevoraussetzungsparameter (im Beispiel aus Tabelle 1 die beiden Konzentrationen der Farbstoffvorstufen) unter Anwendung der zuvor bestimmten Beziehung (des Modells) Werte für den Färbeergebnisparameter (die Helligkeit L*) bestimmen.

In verschiedenen Ausführungsbeispielen kann in Knoten 18 ein so genannter Numerischer Bewerter 250 bereitgestellt sein, welcher unter Verwendung der mittels des Weka-Vorhersagers 240 bestimmten Werte für den Färbeergebnisparameter und der/des tatsächlich gemessenen Färbeergebnisparameter/s Werte berechnet, welche als Maß für eine Anpassungsgüte des Modells genutzt werden können. Beispielsweise können ein Bestimmtheitsmaß $R^2$ (welches für eine lineare Regression einem quadrierten Korrelationskoeffizienten entspricht; generell können gute Werte für das Bestimmtheitsmaß $R^2$ etwa zwischen 0,9 und 1,0 liegen), ein mittlerer absoluter Fehler, ein mittlerer quadratischer Fehler, eine Standardabweichung und/oder eine mittlere absolute Abweichung bestimmt werden. Besonderes Augenmerk kann in verschiedenen Ausführungsbeispielen auf das Bestimmtheitsmaß $R^2$ und auf die mittlere absolute Abweichung gelegt werden.

Ein solches Verfahren zum computergestützten Ermitteln von Eigenschaften von Haarfarben gemäß verschiedenen Ausführungsbeispielen wurde unter Verwendung der 14 Farbstoffvorstufen als Färbevoraussetzungsparameter und der Helligkeit L der Haarfarbe als Färbeergebnisparameter ausgeführt.

Figur 3 zeigt ein Diagramm 300, welches ein Ergebnis des Verfahrens zum computergestützten Ermitteln von Eigenschaften von Haarfarben darstellt.

Das Modell selbst ist nicht darstellbar, denn das würde eine 15-dimensionale Darstellung erfordern. Deshalb sind in Figur 3 die modellierten (vorhergesagten) Helligkeitswerte L in Abhängigkeit von den gemessenen Helligkeitswerten aufgetragen. Im Idealfall würden die Datenpunkte auf einer Linie mit einer Steigung von 1 liegen.

Die beispielhaft ermittelten Werte für die Anpassungsgüte sind dabei $R^2$ = 0.436, mittlerer absoluter Fehler = 3,704, mittlerer quadratischer Fehler: 21,542, Standardabweichung 4,641 und mittlere absolute Abweichung 0.

Auch wenn das Bestimmtheitsmaß $R^2$ in diesem Beispiel schwach sein kann und der mittlere absolute Fehler mit 3,7 recht hoch sein kann, können mit Hilfe eines relativ einfachen Modells (lineare Regression) einigermaßen brauchbare Werte für Eigenschaften (Helligkeitswerte) unbekannter Färbemittelrezepturen bestimmt werden. Zur Bestimmung von Helligkeitswerten für unbekannte Färbemittelrezepturen können diese in den Vorhersageknoten, auch als Prädiktorknoten bezeichnet, eingespeist werden.

In Figur 4 ist ein Ablaufschema 400 dargestellt, welches funktionelle Komponenten und ihr Zusammenwirken bei einem Ausführen eines Verfahrens zum computergestützten Ermitteln von Eigenschaften von Haarfarben gemäß verschiedenen Ausführungsbeispielen darstellt.

Das in Figur 4 dargestellte Ablaufschema 400 kann in Vielem dem Ablaufschema 200 auf Fig.2 entsprechen. Beispielsweise kann der XLS Leser 210 in Knoten 1 der gleiche sein wie der XLS Leser 210 aus Fig.2. Ebenso kann der Spaltenfilter 220 in Knoten 2 der gleiche sein wie der Spaltenfilter 220 aus Fig.2.

In Knoten 23 kann gemäß verschiedenen Ausführungsbeispielen eine funktionelle Komponente 330 zum Bestimmen einer Beziehung zwischen den Färbevoraussetzungsparametern (den 14 Farbstoffvorstufen) und des Färbeergebnisparameters (der Helligkeit der Haarfarbe L*) mittels eines Baum Ensemble Lerners bereitgestellt sein.

Ein Baum Ensemble Lerner (auch als Tree Ensemble Learner oder als Random Forest (geschützter Begriff) bezeichnet), welcher für eine kategoriale Klassifikation oder eine Regression eingesetzt werden kann, kann als einer der leistungsfähigsten Algorithmen im Gebiet der Prädiktiven Analytik betrachtet werden.

Allgemein kann mit Random Forest ein Klassifikationsverfahren bezeichnet werden, welches aus mehreren verschiedenen, unkorrelierten Entscheidungsbäumen besteht. Alle Entscheidungsbäume können unter einer bestimmten Art von Randomisierung während eines Lernprozesses gewachsen sein. Für eine Klassifikation kann jeder Baum in diesem Wald eine Entscheidung treffen dürfen, und die Klasse mit den meisten Stimmen kann die endgültige Klassifikation entscheiden. Neben einer Klassifikation kann der Random Forest auch zu einer Regression eingesetzt werden.

Ein von dem Random Forest (dem Baum Ensemble Lerner) ausgeführtes Verfahren zum Auffinden der Beziehung zwischen den Färbevoraussetzungsparametern und dem Färbeergebnisparameter kann anschaulich beschrieben werden als ein Auffinden von Beziehungen zwischen verschiedenen oder teilweise überlappenden Teilmengen der ausgewählten Daten. Aus den gefundenen Beziehungen für die Teilmengen wird eine Beziehung für die gesamten bei Knoten 2 ausgewählten Daten bestimmt oder erstellt, welche die meisten der Beziehungen für die Teilmengen am besten repräsentiert. Die Beziehungen für die Teilmengen können beispielsweise mittels Regression (z.B. linear oder polynom) bestimmt werden. Diese gefundene Beziehung kann auch als Modell bezeichnet werden.

Im Beispiel wurden für eine Konfiguration des Baum Ensemble Lerners Standardwerte der Software KNIME 2.11.2 verwendet.

Das Anwenden des Baum Ensemble Lerners 330 kann nach dem Auswählen (in Knoten 2) der für die das Bestimmen der Beziehung zu verwendenden Spalten ausgeführt werden.

In verschiedenen Ausführungsbeispielen kann in Knoten 24 ein so genannter Baum Ensemble Vorhersager 440 bereitgestellt sein, welchem sowohl die Ergebnisse der Modellerstellung (des Bestimmens der Beziehung) aus Knoten 23 als auch die ungenutzten Haarfarben-Daten aus Knoten 2 zugeführt werden können. Der Baum Ensemble Vorhersager 440 kann für sämtliche ausgewählten Färbevoraussetzungsparameter (im Beispiel aus Tabelle 1 die beiden Konzentrationen der Farbstoffvorstufen) unter Anwendung der zuvor bestimmten Beziehung (des Modells) Werte für den Färbeergebnisparameter (die Helligkeit L*) bestimmen.

In verschiedenen Ausführungsbeispielen kann in Knoten 22 ein Numerischer Bewerter 450 (ähnlich dem Numerischen Bewerter 250 aus Fig.2) bereitgestellt sein, welcher unter Verwendung der mittels des Baum Ensemble Vorhersagers 440 bestimmten Werte für die Färbeergebnisparameter und der/n tatsächlich gemessenen Färbeergebnisparameter/n Werte berechnet, welche als Maß für eine Anpassungsgüte des Modells genutzt werden können. Beispielsweise können ein Bestimmtheitsmaß $R^2$, ein mittlerer absoluter Fehler, ein mittlerer quadratischer Fehler, eine Standardabweichung und/oder eine mittlere absolute Abweichung bestimmt werden. Besonderes Augenmerk kann in verschiedenen Ausführungsbeispielen auf das Bestimmtheitsmaß $R^2$ und auf die mittlere absolute Abweichung gelegt werden.

Ein solches Verfahren zum computergestützten Ermitteln von Eigenschaften von Haarfarben gemäß verschiedenen Ausführungsbeispielen wurde unter Verwendung der 14 Farbstoffvorstufen als Färbevoraussetzungsparameter und der Helligkeit L der Haarfarbe als Färbeergebnisparameter Z ausgeführt.

Figuren 5A und 5B zeigen zwei Diagramme 500 und 501, welche auf unterschiedliche Weise Ergebnisse dieses Verfahrens darstellen.

In Fig.5A sind die modellierten (vorhergesagten) Helligkeitswerte L in Abhängigkeit von den gemessenen Helligkeitswerten aufgetragen. Im Idealfall würden die Datenpunkte auf einer Linie mit einer Steigung von 1 liegen.

In Fig.5B sind Residuen, also absolute Abweichungen zwischen den modellierten und den gemessenen Helligkeitswerten, als Säulendiagramm dargestellt.

Die beispielhaft ermittelten Werte für die Anpassungsgüte sind dabei $R^2$ = 0.939, mittlerer absoluter Fehler = 1,073, mittlerer quadratischer Fehler = 2,337, Standardabweichung = 1,529 und mittlere absolute Abweichung -0,06.

Gegenüber der im Zusammenhang mit Fig.2 und Fig.3 beschriebenen Beispiel, bei welchem eine lineare Regression für das Bestimmen der Beziehung verwendet wurde, zeigen sich in diesem Beispiel bei Verwendung des Baum Ensembles deutlich verbesserte Werte, beispielsweise ein Bestimmtheitsmaß $R^2$ = 0.939, welches wie erwünscht zwischen 0,9 und 1 liegt, und ein mittlerer absoluter Fehler der Helligkeit (Luminosity) von ca. 1, was unterhalb der Wahrnehmungsschwelle liegt, so dass berechnete Werte visuell nicht mehr von experimentell bestimmten Werten unterschieden werden könnten.

Nicht in den Figuren dargestellt ist eine beispielhafte Modellierung von a* und b*-Werten mittels des Baum Ensemble Lerners.

Dafür wird das Verfahren zum computergestützten Ermitteln von Eigenschaften von Haarfarben im Wesentlichen genauso durchgeführt wie oben zur Bestimmung der Helligkeit L* beschrieben, mit dem Unterschied, dass bei dem Spaltenfilter 220 nicht die Spalte mit der Helligkeit L* zum Färbeergebnisparameter bestimmt wird, sondern ein Rot/Grün-Parameter a* bzw. ein Blau/Gelb-Parameter b*.

Bei einer Bestimmung einer Anpassungsgüte mittels des Numerischen Bewerters 450 in Knoten 22 ergeben sich die folgenden Werte, welche eine ähnliche Anpassungsgüte (Genauigkeit) wie die Werte für das Modell für L* aufweisen: Die beispielhaft ermittelten Werte für a* sind $R^2$ = 0.959, mittlerer absoluter Fehler = 1,236, mittlerer quadratischer Fehler = 4,067, Standardabweichung = 2,017 und mittlere absolute Abweichung -0,115.

Die beispielhaft ermittelten Werte für b* sind $R^2$ = 0.94, mittlerer absoluter Fehler = 1,366, mittlerer quadratischer Fehler = 4,763, Standardabweichung = 2,183 und mittlere absolute Abweichung - 0,063.

Anstelle einer linearen Regression oder eines Baum Ensemble Lerners können in verschiedenen Ausführungsbeispielen allerdings auch andere Verfahren eingesetzt werden, beispielsweise Support Vector Machines oder Neuronale Netze.

Bei einem beispielhaft ausgeführten Verfahren zum computergestützten Ermitteln von Eigenschaften von Haarfarben unter Verwendung eines Neuronalen Netzes (verwendet wurde das Modell Multi Layer Perceptron mit Standardparametern) wurden in Fig.6A und Fig.6B dargestellte Ergebnisse erzielt.

Figur 6A und 6B zeigen zwei Diagramme 600 und 601, welche auf unterschiedliche Weise diese Ergebnisse des Verfahrens zum computergestützten Ermitteln von Eigenschaften von Haarfarben darstellen.

In Fig.6A sind die modellierten (vorhergesagten) Helligkeitswerte L in Abhängigkeit von den gemessenen Helligkeitswerten aufgetragen. Im Idealfall würden die Datenpunkte auf einer Linie mit einer Steigung von 1 liegen.

In Fig.6B sind Residuen, also absolute Abweichungen zwischen den modellierten und den gemessenen Helligkeitswerten als Säulendiagramm (also eine Häufigkeitsverteilung der Residuen) dargestellt.

Die Anpassungsgüte ist auch hier besser als im Fall der linearen Regression, allerdings ein wenig schlechter als im Fall des Tree-Ensemble Lerners.

Figur 7 zeigt ein Diagramm 700, welches ein Ergebnis eines Verfahrens zum computergestützten Ermitteln von Eigenschaften von Haarfarben gemäß einem Ausführungsbeispiel darstellt.

Im vorliegenden Beispiel wurde das Verfahren im Wesentlichen so ausgeführt wie es im Zusammenhang mit Fig.4, Fig.5A und Fig.5B beschrieben wurde, mit dem Unterschied, dass als Färbeergebnisparameter anstelle der Helligkeit L* der Haarfarbe die Haarfarbe selbst in Form der drei Parameter L*, a* und b* als Färbeergebnisparameter ausgewählt wurden (mittels des Spaltenfilters 220). Die Bestimmung des Modells (mittels des Baum Ensemble Lerners 430) erfolgte somit für alle drei Färbeergebnisparameter L*, a* und b* gleichzeitig.

Als Ergebnis des Ausführungsbeispiels ist in Fig.7 ein Säulendiagramm für einen (vorzeichenlosen) Farbabstand $\Delta E$, in welchen die Unterschiede in den drei Einzelgrößen L*, a* und b* für eine Berechnung eines euklidischen Abstands einfließen, zwischen der ermittelten Farbe und der gemessenen Farbe dargestellt. Dabei ist für 75% der Werte $\Delta E < 2{,}5$.

Figur 8 zeigt ein Diagramm 800, welches ein Ergebnis eines Verfahrens zum computergestützten Ermitteln von Eigenschaften von Haarfarben gemäß einem Ausführungsbeispiel darstellt.

Im vorliegenden Beispiel wurde das Verfahren im Wesentlichen so ausgeführt wie es im Zusammenhang mit Fig.4, Fig.5A und Fig.5B beschrieben wurde, mit dem Unterschied, dass als Färbeergebnisparameter anstelle der Helligkeit L* der Haarfarbe eine Waschechtheit nach 24 Haarwäschen (nicht in Tabelle 1 oder Tabelle 2 dargestellt) verwendet wird. Als Maß für die Waschechtheit kann ein Farbabstand $\Delta E$ (bezeichnet als $\Delta E_{00}$) genutzt werden. In Fig.8 ist der gemäß dem bestimmten Modell berechnete Farbabstand gegenüber dem experimentell gemessenen Farbabstand aufgetragen, wobei die Werte wie gewünscht im Wesentlichen um eine Gerade mit der Steigung 1 streuen.

Numerische Ergebnisse für eine Anpassungsgüte eines weiteren Ausführungsbeispiels, bei welchem der Farbabstand nach 12 Haarwäschen (siehe Tabelle 2) als Färbeergebnisparameter verwendet wurde (wiederum unter Verwendung eines Baum Ensemble Lerners, wie oben im Zusammenhang mit Fig.4 und Fig. 5A und 5B beschrieben) sind $R^2 = 0.883$, mittlerer absoluter Fehler = 0,22, mittlerer quadratischer Fehler = 0,124, Standardabweichung = 0,353 und mittlere absolute Abweichung -0,01. Hier liegt der mittlere absolute Fehler, welcher einem mittleren $\Delta(\Delta E)$ zwischen gemessenen und berechneten Waschechtheiten entspricht, liegt mit $\Delta(\Delta E)=0.22$ weit unterhalb jeglicher visueller Differenzierbarkeit.

Figur 9 zeigt ein Diagramm 900, welches ein Ergebnis eines Verfahrens zum computergestützten Ermitteln von Eigenschaften von Haarfarben gemäß einem Ausführungsbeispiel darstellt.

Im vorliegenden Beispiel wurde das Verfahren im Wesentlichen so ausgeführt wie es im Zusammenhang mit Fig.4, Fig.5A und Fig.5B beschrieben wurde, mit dem Unterschied, dass als Färbeergebnisparameter anstelle der Helligkeit L* der Haarfarbe eine gemessene Grauabdeckung verwendet wird.

Figuren 10A, 10B und 10C zeigen drei Diagramme, welche das Verhältnis von Messpunkten, wahrer Funktion und Modell für verschiedene bei einem Verfahren zum computergestützten Ermitteln von Eigenschaften von Haarfarben gemäß verschiedenen Ausführungsbeispielen verwendeten Modellen darstellen.

Hier zeigt sich, dass das Verfahren zum computergestützten Ermitteln von Eigenschaften von Haarfarben zwar typischerweise ein Modell bereitstellen wird, das Modell aber nicht unbedingt eine gute Repräsentation der Haarfarben-Daten darstellt. Für ein gutes Modell kann in verschiedenen Ausführungsbeispielen ein großer Datensatz gewählt werden, der es ermöglicht, Trainings-Datensätze und Validierungsdatensätze aus dem Datensatz auszuwählen.

Die im Zusammenhang mit den Figuren 2 bis 9 dargestellten Ausführungsbeispiele sollen lediglich einer Veranschaulichung dienen. Das Verfahren zum computergestützten Ermitteln von Eigenschaften von Haarfarben kann gemäß verschiedenen Ausführungsbeispielen gegenüber den obigen Beispielen an vielen Stellen variiert oder erweitert werden. Beispielsweise können die verwendeten Färbevoraussetzungsparameter und Färbeergebnisparameter geändert, erweitert oder beschränkt werden, Algorithmen zur Bestimmung der Beziehung zwischen den Färbevoraussetzungsparametern und der/den Färbeergebnisparameter kann variiert werden, Parameter für die Bestimmung der Beziehung können, beispielsweise abweichen von den von KNIME vorgegebenen Standardparametern bestimmt werden, beispielsweise verschiedene Färbevoraussetzungsparameter oder Färbeergebnisparameter mit verschiedenen Gewichtungen versehen werden, es kann ein anderes Computerprogramm zum Ausführen des Verfahrens genutzt werden, oder ähnliches.

Zusätzlich ist es möglich, die Daten in Test-, Trainings- und Validierungssätze aufzuteilen.

Die Figuren 10A bis 10C veranschaulichen, dass beispielsweise ein Grad einer anzupassenden Funktion beschränkt werden kann, denn obwohl das Verfahren zum computergestützten Ermitteln von Eigenschaften von Haarfarben kann gemäß verschiedenen Ausführungsbeispielen auch für Funktionen ersten (Fig.10A) und 5. Grades (Fig.10C) ein Ergebnis liefern würde, würde ein Vergleich von ermittelten Anpassungsgüten der drei Modelle (Beziehungen) ergeben, dass ein Modell unter Verwendung einer Funktion vierten Grades das beste Ergebnis von den drei Modellen liefert.

Figur 11 zeigt ein Ablaufdiagramm, welches ein Verfahren zum computergestützten Ermitteln von Eigenschaften von Haarfarben gemäß verschiedenen Ausführungsbeispielen darstellt.

In verschiedenen Ausführungsbeispielen kann das Verfahren zum computergestützten Ermitteln von Eigenschaften von Haarfarben aufweisen ein Bereitstellen von Haarfarben-Daten, wobei die Haarfarben-Daten für eine Mehrzahl von Färbevorgängen jeweils Werte für eine Mehrzahl von Färbevoraussetzungsparametern und für mindestens einen Färbeergebnisparameter aufweisen, wobei für jeden Färbevorgang der Mehrzahl von Färbevorgängen die Mehrzahl von Färbevoraussetzungsparametern eine erste Konzentration einer ersten Farbstoffvorstufe und eine zweite Konzentration einer zweiten Farbstoffvorstufe aufweist; wobei für jeden Färbevorgang der Mehrzahl von Färbevorgängen der mindestens eine Färbeergebnisparameter eine gemessene Angabe über eine Eigenschaft von Haarfarben aufweist (bei 1110), und ein Ermitteln einer Beziehung zwischen der Mehrzahl von Färbevoraussetzungsparametern und dem mindestens einen Färbeergebnisparameter mittels prädiktiver Analytik basierend auf den Haarfarben-Daten (bei 1120).

Figur 12 ist eine graphische Darstellung 1200 einer Datenverarbeitungsvorrichtung 1210 zum computergestützten Ermitteln von Eigenschaften von Haarfarben gemäß verschiedenen Ausführungsbeispielen.

Die Datenverarbeitungsvorrichtung 1200 kann beispielsweise ein PC, ein Laptop oder eine sonstige beliebige Datenverarbeitungsvorrichtung sein oder aufweisen, welche geeignet ist, das Verfahren zum computergestützten Ermitteln von Eigenschaften von Haarfarben auszuführen, also beispielsweise einen hinreichend großen Speicher und einen hinreichend leistungsfähigen Prozessor aufweist.

Die Datenverarbeitungsvorrichtung 1200 weist einen Prozessor 1220 auf. Der Prozessor 1220 kann beispielsweise ein Mikroprozessor der Datenverarbeitungsvorrichtung 1200 sein oder einen solchen Mikroprozessor aufweisen.

In verschiedenen Ausführungsbeispielen kann die Datenverarbeitungsvorrichtung 1200 eine Datenspeichervorrichtung 1230 aufweisen. Die Datenspeichervorrichtung kann ein interner oder externer Datenspeicher 1230 einer der genannten Datenverarbeitungsvorrichtungen 1200 sein oder einen solchen Datenspeicher 1230 aufweisen. Der Datenspeicher 1230 kann eingerichtet sein, Daten zu speichern, welche bei einer Durchführung des Verfahrens zum computergestützten Ermitteln von Eigenschaften von Haarfarben gespeichert und/oder abgerufen werden, beispielsweise die Haarfarben-Daten.

In verschiedenen Ausführungsbeispielen kann die Datenverarbeitungsvorrichtung 1200 eine Anzeigevorrichtung 1240 aufweisen. Die Anzeigevorrichtung 1240 kann beispielsweise einen Bildschirm eines PCs, eines Laptops oder einer sonstigen beliebigen Datenverarbeitungsvorrichtung 1200 aufweisen. Die Anzeigevorrichtung kann beispielsweise genutzt werden, um Ergebnisse des Verfahrens zum computergestützten Ermitteln von Eigenschaften von Haarfarben darzustellen, Eingabeparameter für das Ausführen des Verfahrens zu erfragen, oder ähnliches Die Nutzung der Anzeigevorrichtung kann insbesondere in einem Verkaufsort ("point of sale") für Haarbehandlungsprodukte erfolgen.

In verschiedenen Ausführungsbeispielen kann die Datenverarbeitungsvorrichtung 1200 eine Eingabevorrichtung 1250 zum Bereitstellen von Informationen an die Datenverarbeitungsvorrichtung 1200 aufweisen, beispielsweise eine Tastatur, eine Maus, eine berührungsempfindliche Oberfläche der Anzeigevorrichtung 1240, oder ähnliches.

Weitere vorteilhafte Ausgestaltungen des Verfahrens ergeben sich aus der Beschreibung der Vorrichtung und umgekehrt.

## Patentansprüche

1. Verfahren zum computergestützten Ermitteln von Eigenschaften von Haarfarben, aufweisend:

    • Bereitstellen von Haarfarben-Daten geeignet zur Anwendung von prädiktiver Analytik,

        ◦ wobei die Haarfarben-Daten für eine Mehrzahl von Färbevorgängen jeweils Werte für eine Mehrzahl von Färbevoraussetzungsparametern und für mindestens einen Färbeergebnisparameter aufweisen,
        o wobei für jeden Färbevorgang der Mehrzahl von Färbevorgängen die Mehrzahl von Färbevoraussetzungsparametern eine erste Konzentration einer ersten Farbstoffvorstufe und eine zweite Konzentration einer zweiten Farbstoffvorstufe aufweist, wobei die Mehrzahl von Färbevoraussetzungsparametern ferner eine Grundhaarfarbe aufweist, wobei die Grundhaarfarbe in einem Farbraum parametrisiert ist;
        o wobei für jeden Färbevorgang der Mehrzahl von Färbevorgängen der mindestens eine Färbeergebnisparameter eine gemessene Angabe über eine Eigenschaft von mittels Färbens von Haar erzeugten Haarfarben aufweist, wobei die Eigenschaften von mittels Färbens von Haar erzeugten Haarfarben: einen oder mehr Parameter einer in einem Farbraum parametrisierten Haarfarbe aufweisen;

    • Ermitteln einer Beziehung zwischen der Mehrzahl von Färbevoraussetzungsparametern und dem mindestens einen Färbeergebnisparameter mittels prädiktiver Analytik basierend auf den Haarfarben-Daten, wobei die Beziehung ein Modell umfasst, das für jeden Wert einer Kombination von Werten für eine Mehrzahl von Färbevoraussetzungsparametern, umfassend Werte, die keinen experimentell bestimmten Werten entsprechen, einen Wert für mindestens einen Färbeergebnisparameter ermittelt,

    **dadurch gekennzeichnet, dass** die Mehrzahl von Färbevoraussetzungsparametern ferner eine Vorschädigung des Haars aufweist.

2. Verfahren gemäß Anspruch 1, ferner aufweisend:
    Bestimmen, mittels der ermittelten Beziehung, eines Werts für einen Färbeergebnisparameter zu einer Wertekombination aus jeweils einem Wert für eine ausgewählte Mehrzahl von Färbevoraussetzungsparametern, wobei die Wertekombination für keinen der Färbevorgänge mit dessen Wertekombination für die ausgewählte Mehrzahl von Färbevoraussetzungsparametern identisch ist.

**3.** Verfahren gemäß Anspruch 1 oder 2,
wobei die prädiktive Analytik mindestens ein Verfahren nutzt aus einer Gruppe von Verfahren, wobei die Gruppe von Verfahren aufweist:

lineare oder multi-lineare Regression,
multiple polynome Regression,
Neuronale-Netze-Verfahren,
Support Vector Machine-Verfahren; und
Entscheidungsbäume-Verfahren.

**4.** Verfahren gemäß Anspruch 3,
wobei das Entscheidungsbäume-Verfahren Entscheidungsbaum-Ensembles ("Tree-Ensembles") nutzt.

**5.** Verfahren gemäß einem der Ansprüche 1 bis 4,
wobei die Mehrzahl von Färbevoraussetzungsparametern ferner einen Ergrauungsgrad aufweist.

**6.** Verfahren gemäß einem der Ansprüche 1 bis 5,
wobei der mindestens eine Färbeergebnisparameter mindestens eine weitere Eigenschaft von mittels Färbens von Haar erzeugten Haarfarben aufweist, wobei die weiteren Eigenschaften von mittels Färbens von Haar erzeugten Haarfarben aufweisen:

eine Waschechtheit;
eine Lichtechtheit; und
eine Fähigkeit zur Grauabdeckung.

**7.** Verfahren gemäß einem der Ansprüche 1 bis 6,
wobei der mindestens eine Färbeergebnisparameter eine Mehrzahl von Färbeergebnisparametern aufweist.

**8.** Datenverarbeitungsvorrichtung (1200) zum Ausführen eines computergestützten Ermittelns von Eigenschaften von Haarfarben, umfassend einen Prozessor (1220), der eingerichtet ist, das Verfahren gemäß Anspruch 1 auszuführen.

## Claims

**1.** A method for computer-assisted determination of hair colour characteristics, comprising:

• providing hair colour data suitable for application of predictive analytics,

o wherein the hair colour data for a plurality of colouring processes comprise values for a plurality of colouring prerequisite parameters and for at least one colouring result parameter ,
o wherein, for each dyeing process of the plurality of dyeing processes , the plurality of dyeing prerequisite parameters comprises a first concentration of a first dye precursor and a second concentration of a second dye precursor , wherein the plurality of dyeing prerequisite parameters further comprises a base hair colour, wherein the base hair colour is parameterised in a colour space , wherein the plurality of dyeing prerequisite parameters further comprises prior damage to the hair;
o wherein, for each dyeing process of the plurality of dyeing processes, the at least one dyeing result parameter comprises a measured indication of a property of hair colours produced by dyeing hair, wherein the properties of hair colours produced by dyeing hair comprise: one or more parameters of a hair colour parameterised in a colour space;

• determining a relationship between the plurality of dyeing prerequisite parameters and the at least one dyeing result parameter by means of predictive analytics based on the hair colour data , wherein the relationship comprises a model that, for each value of a combination of values for a plurality of dyeing prerequisite parameters , including values that do not correspond to experimentally determined values, a value for at least one dyeing result parameter is determined .

2. Method according to claim 1, further comprising:
determining, by means of the determined relationship, a value for a dyeing result parameter for a combination of values, each comprising a value for a selected plurality of dyeing prerequisite parameters, wherein the combination of values is not identical for any of the dyeing processes with its combination of values for the selected plurality of dyeing prerequisite parameters.

3. Method according to claim 1 or 2,
wherein the predictive analytics uses at least one method from a group of methods, wherein the group of methods comprises:

linear or multi-linear regression,
multiple polynomial regression,
neural network methods,
support vector machine methods; and
decision tree methods.

4. Method according to claim 3,
wherein the decision tree method uses decision tree ensembles ("tree ensembles").

5. Method according to one of claims 1 to 4,
wherein the plurality of colouring prerequisite parameters further comprises a degree of greying.

6. Method according to one of claims 1 to 5,
wherein the at least one dyeing result parameter comprises at least one further property of hair colours produced by dyeing hair, wherein the further properties of hair colours produced by dyeing hair comprise :

a wash fastness;
light fastness; and
an ability to cover grey hair.

7. Method according to any one of claims 1 to 6,
wherein the at least one dyeing result parameter comprises a plurality of dyeing result parameters.

8. Data processing apparatus for performing computer-aided determination of characteristics of hair colours, wherein the data processing apparatus is configured to perform the method according to claim 1.

## Revendications

1. **Procédé** pour déterminer par ordinateur les propriétés des couleurs de cheveux, comprenant :

• la fourniture de données de couleur de cheveux adaptées à l'application d'une analyse prédictive,

o les données de couleur de cheveux pour une pluralité d'opérations de coloration comprenant respectivement des valeurs pour une pluralité de paramètres de conditions préalables à la coloration et pour au moins un paramètre de résultat de coloration ,
o dans lequel, pour chaque processus de coloration de la pluralité de processus de coloration , la pluralité de paramètres de conditions préalables à la coloration comprend une première concentration d'un premier précurseur de colorant et une deuxième concentration d'un deuxième précurseur de colorant , la pluralité de paramètres de condition préalable à la coloration comprenant en outre une couleur de cheveux de base, la couleur de cheveux de base étant paramétrée dans un espace colorimétrique , la pluralité de paramètres de condition préalable à la coloration comprenant en outre une détérioration préalable des cheveux ;
o dans lequel, pour chaque processus de coloration parmi la pluralité de processus de coloration, le au moins un paramètre de résultat de coloration comprend une indication mesurée d'une propriété des couleurs de cheveux produites par la coloration des cheveux, les propriétés des couleurs de cheveux produites par la coloration des cheveux comprenant : un ou plusieurs paramètres d'une couleur de cheveux paramétrée dans un espace colorimétrique ;

• détermination d’une relation entre la pluralité de paramètres de conditions préalables à la coloration et le au moins un paramètre de résultat de coloration au moyen d’une analyse prédictive basée sur les données de couleur de cheveux , , la relation comprenant un modèle qui, pour chaque valeur d’une combinaison de valeurs pour une pluralité de paramètres de conditions préalables à la coloration , y compris des valeurs qui ne correspondent pas à des valeurs déterminées expérimentalement, détermine une valeur pour au moins un paramètre de résultat de coloration t.

**2.** Procédé selon la revendication 1, comprenant en outre :
la détermination, au moyen de la relation déterminée, d’une valeur pour un paramètre de résultat de coloration pour une combinaison de valeurs comprenant chacune une valeur pour une pluralité sélectionnée de paramètres de condition de coloration, la combinaison de valeurs n’étant identique pour aucun des processus de coloration à sa combinaison de valeurs pour la pluralité sélectionnée de paramètres de condition de coloration.

**3.** Procédé selon la revendication 1 ou 2,
dans lequel l’analyse prédictive utilise au moins un procédé parmi un groupe de procédés, le groupe de procédés comprenant :

une régression linéaire ou multilinéaire,
une régression polynomiale multiple,
procédé de réseaux neuronaux,
des procédés de machines à vecteurs de support ; et
des procédés d’arbres de décision.

**4.** Procédé selon la revendication 3,
dans lequel le procédé d’arbres de décision utilise des ensembles d’arbres de décision (« ensembles d’arbres »).

**5.** Procédé selon l’une des revendications 1 à 4,
dans lequel la pluralité de paramètres de condition de coloration comprend en outre un degré de grisaillement.

**6.** Procédé selon l’une des revendications 1 à 5,
dans lequel le au moins un paramètre de résultat de coloration comprend au moins une autre propriété des couleurs de cheveux obtenues par coloration des cheveux, les autres propriétés des couleurs de cheveux obtenues par coloration des cheveux comprenant :

une résistance au lavage ;
une solidité à la lumière ; et
une capacité à couvrir les cheveux gris.

**7.** Procédé selon l’une des revendications 1 à 6,
dans lequel le au moins un paramètre de résultat de teinture comprend une pluralité de paramètres de résultat de teinture.

**8.** Dispositif de traitement de données pour effectuer une détermination assistée par ordinateur des propriétés des couleurs de cheveux, le dispositif de traitement de données étant conçu pour exécuter le procédé selon la revendication 1.

FIG. 1

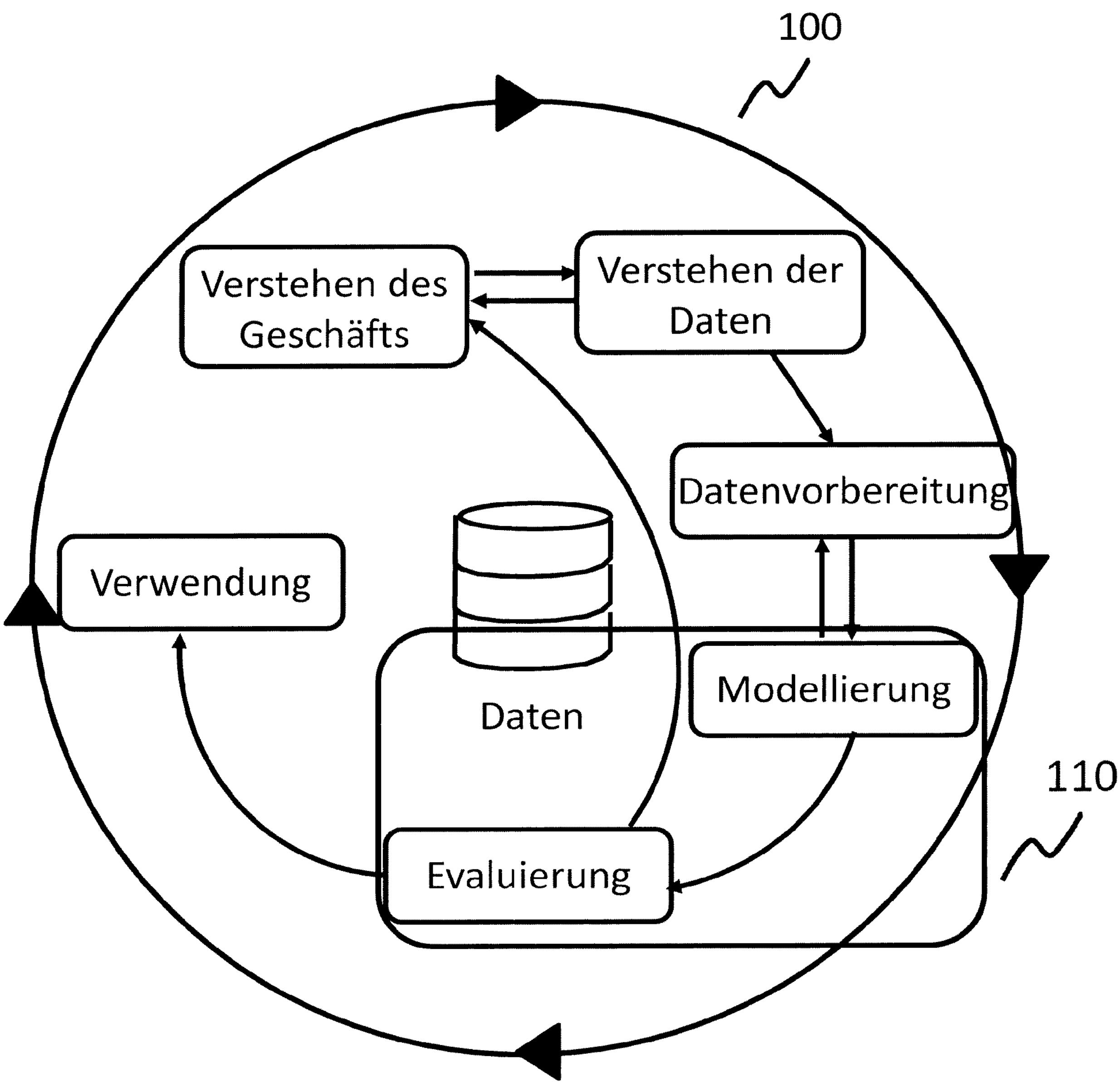
100
Verstehen des Geschäfts
Verstehen der Daten
Datenvorbereitung
Verwendung
Daten
Modellierung
110
Evaluierung

FIG. 2

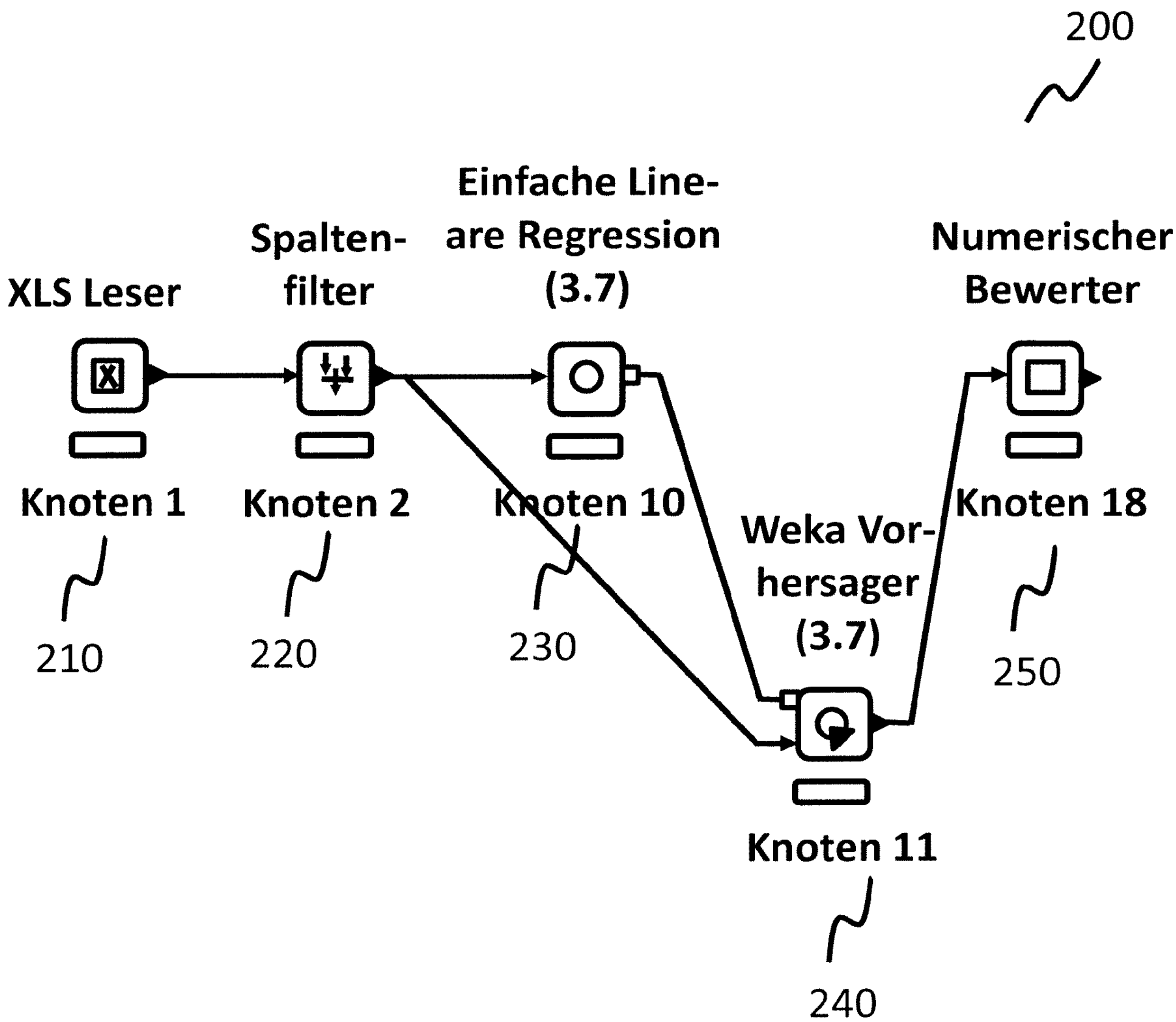
200
XLS Leser
Spalten-
filter
Einfache Line-
are Regression
(3.7)
Numerischer
Bewerter
Knoten 1
Knoten 2
Knoten 10
Weka Vor-
hersager
(3.7)
Knoten 18
Knoten 11
210
220
230
250
240

FIG. 3

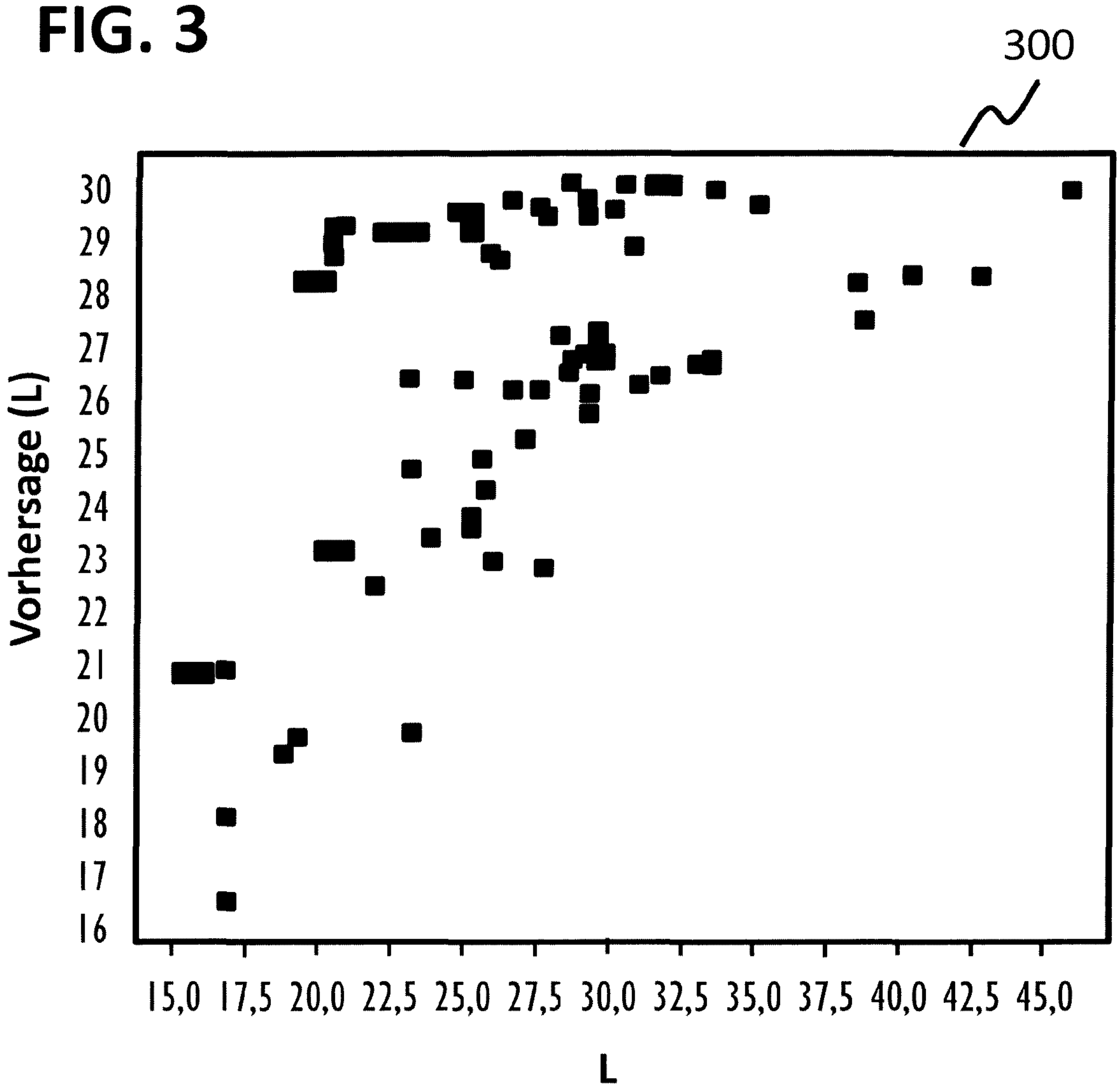
300
Vorhersage (L)
30
29
28
27
26
25
24
23
22
21
20
19
18
17
16
15,0 17,5 20,0 22,5 25,0 27,5 30,0 32,5 35,0 37,5 40,0 42,5 45,0
L

FIG. 4

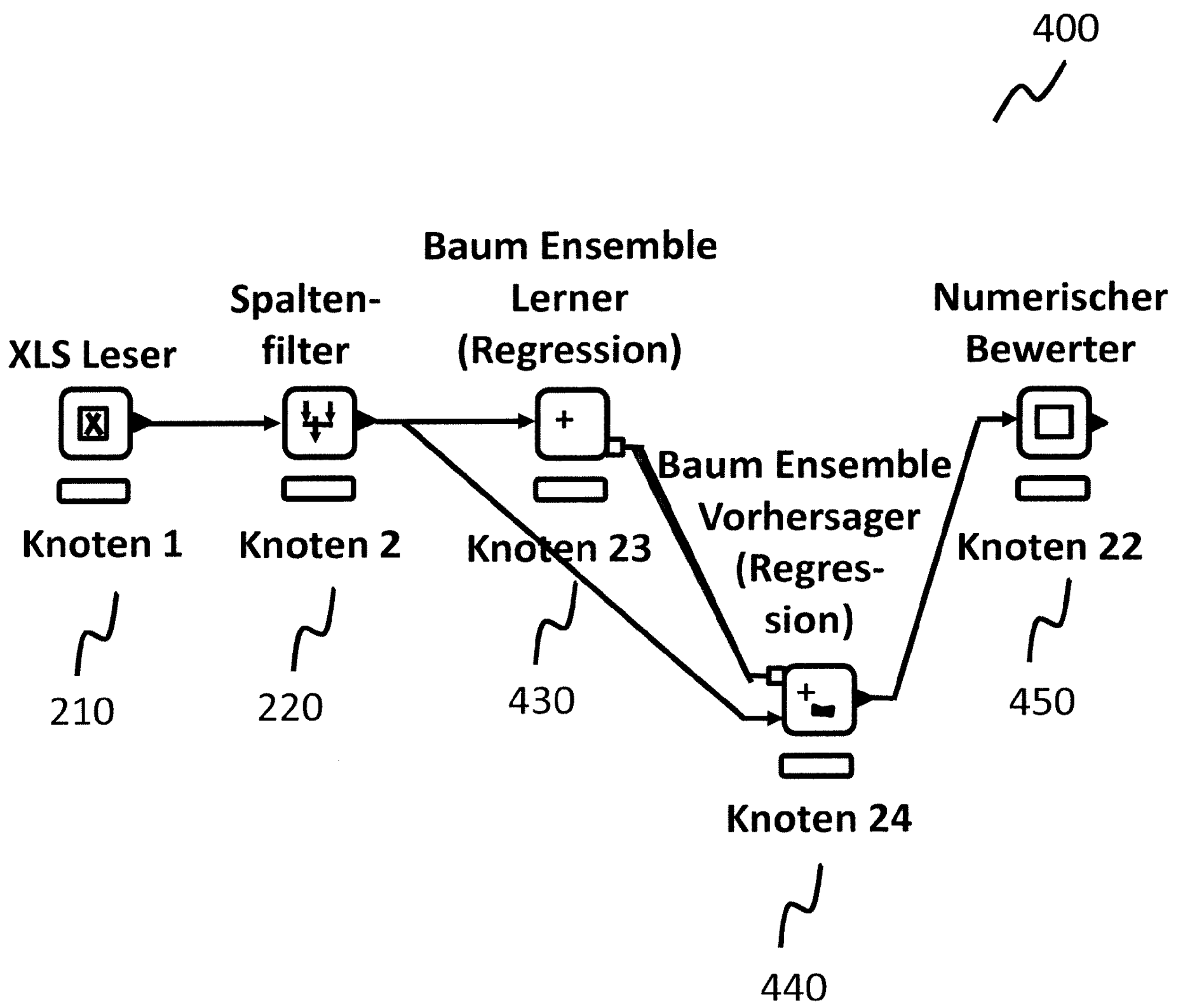
400
XLS Leser
Spalten-
filter
Baum Ensemble
Lerner
(Regression)
Numerischer
Bewerter
Baum Ensemble
Vorhersager
(Regres-
sion)
Knoten 1
Knoten 2
Knoten 23
Knoten 22
Knoten 24
210
220
430
450
440

# FIG. 5A

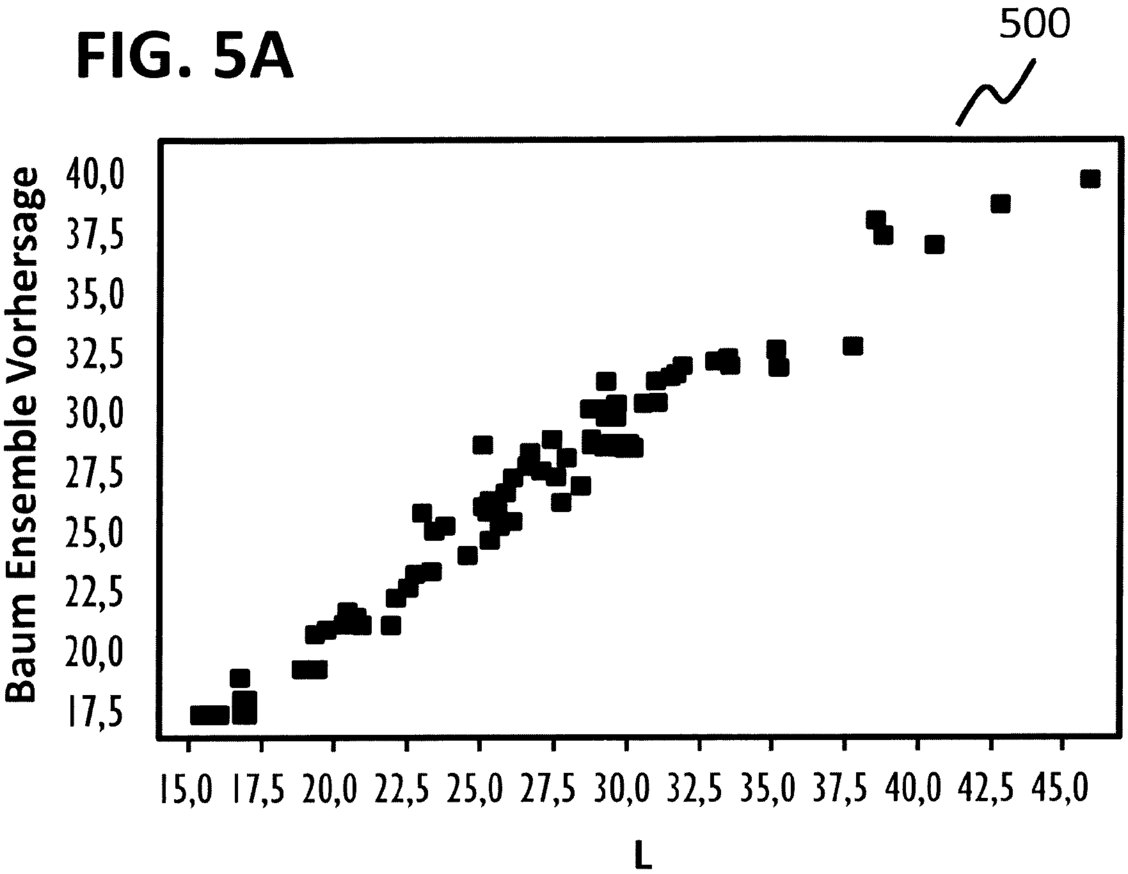
500
Baum Ensemble Vorhersage
40,0
37,5
35,0
32,5
30,0
27,5
25,0
22,5
20,0
17,5
15,0 17,5 20,0 22,5 25,0 27,5 30,0 32,5 35,0 37,5 40,0 42,5 45,0
L

# FIG. 5B

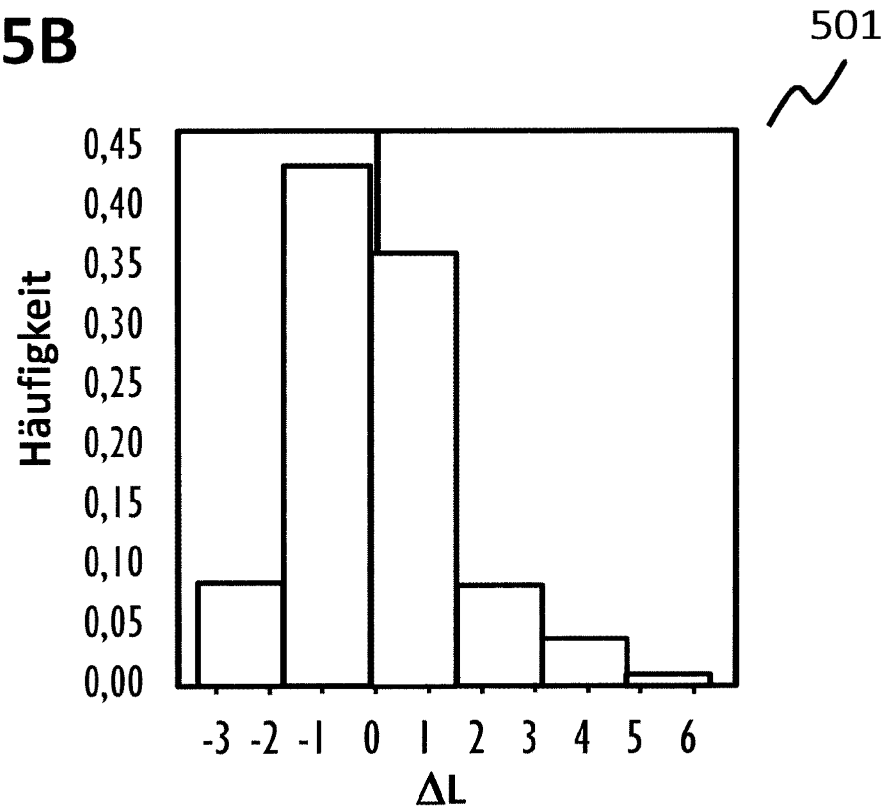
501
Häufigkeit
0,45
0,40
0,35
0,30
0,25
0,20
0,15
0,10
0,05
0,00
-3 -2 -1 0 1 2 3 4 5 6
ΔL

FIG. 6A

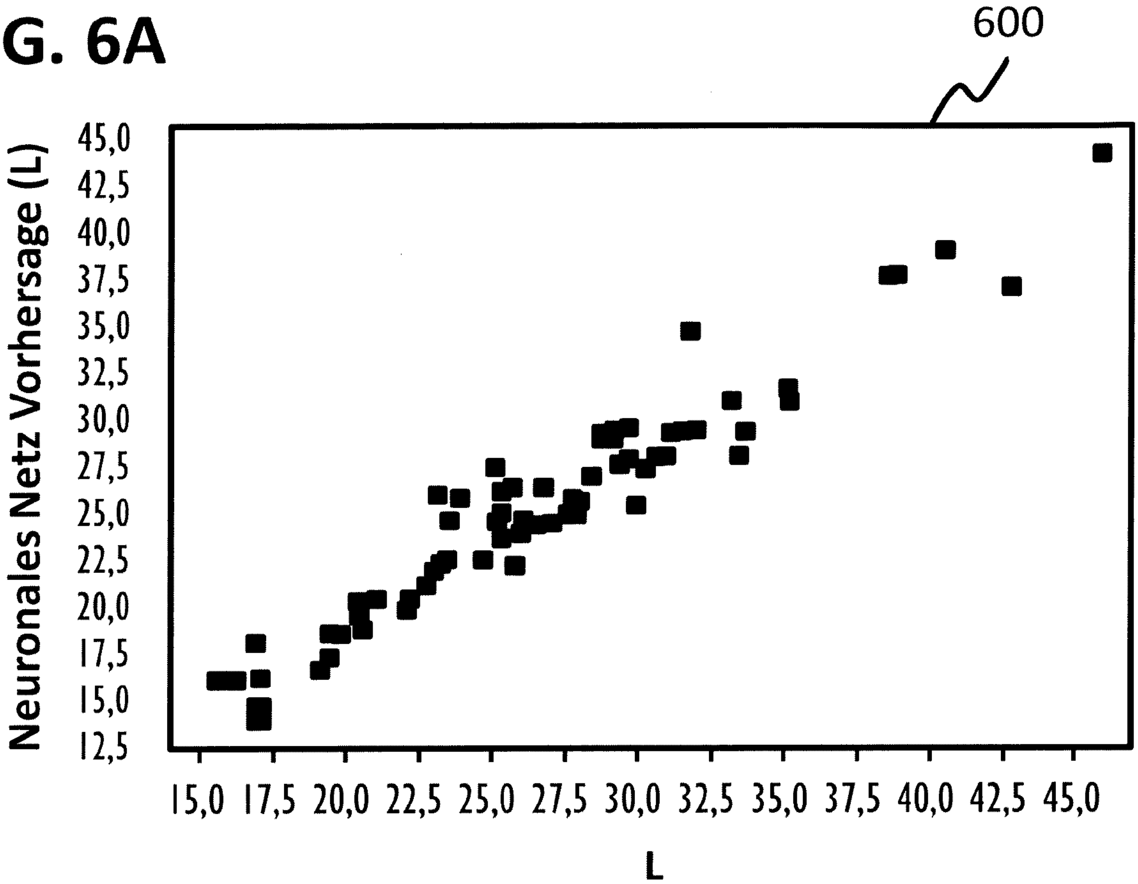
600
Neuronales Netz Vorhersage (L)
45,0
42,5
40,0
37,5
35,0
32,5
30,0
27,5
25,0
22,5
20,0
17,5
15,0
12,5
15,0 17,5 20,0 22,5 25,0 27,5 30,0 32,5 35,0 37,5 40,0 42,5 45,0
L

FIG. 6B

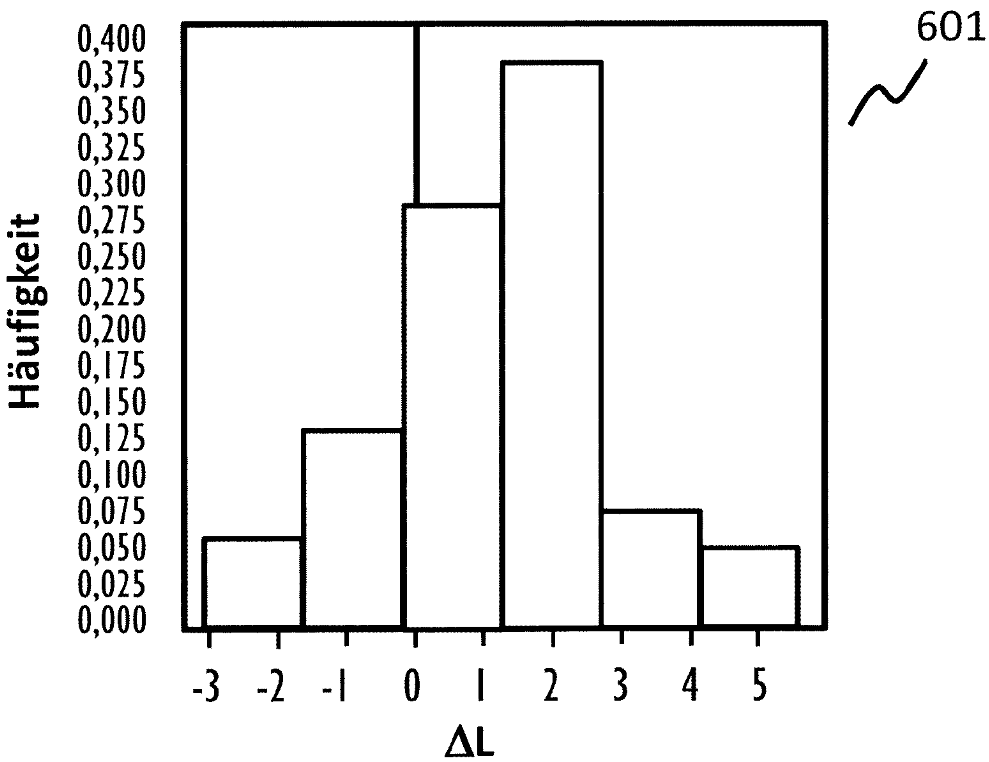
601
Häufigkeit
0,400
0,375
0,350
0,325
0,300
0,275
0,250
0,225
0,200
0,175
0,150
0,125
0,100
0,075
0,050
0,025
0,000
-3 -2 -1 0 1 2 3 4 5
ΔL

FIG. 7

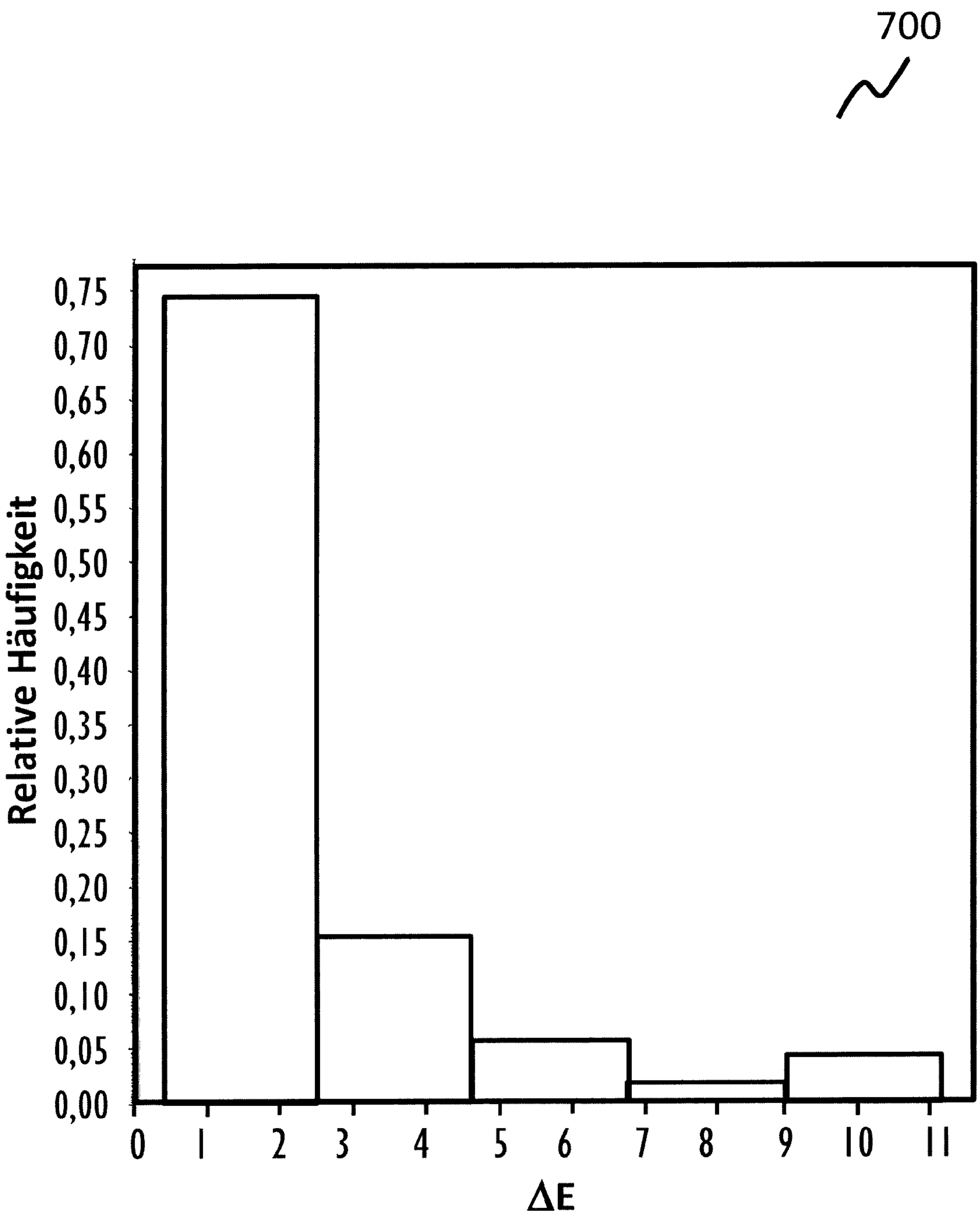
700
Relative Häufigkeit
0,75
0,70
0,65
0,60
0,55
0,50
0,45
0,40
0,35
0,30
0,25
0,20
0,15
0,10
0,05
0,00
0 1 2 3 4 5 6 7 8 9 10 11
ΔE

FIG. 8

800

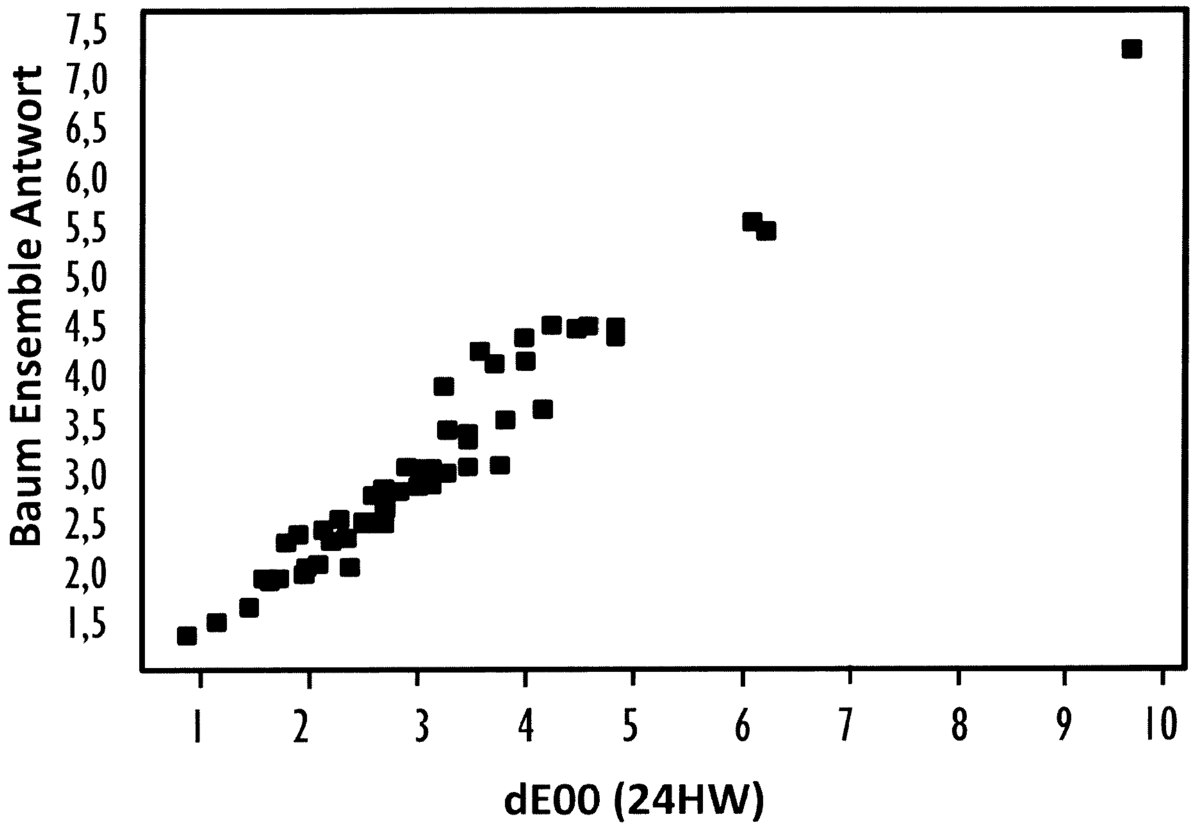
Baum Ensemble Antwort
7,5
7,0
6,5
6,0
5,5
5,0
4,5
4,0
3,5
3,0
2,5
2,0
1,5
1
2
3
4
5
6
7
8
9
10
dE00 (24HW)

FIG. 9

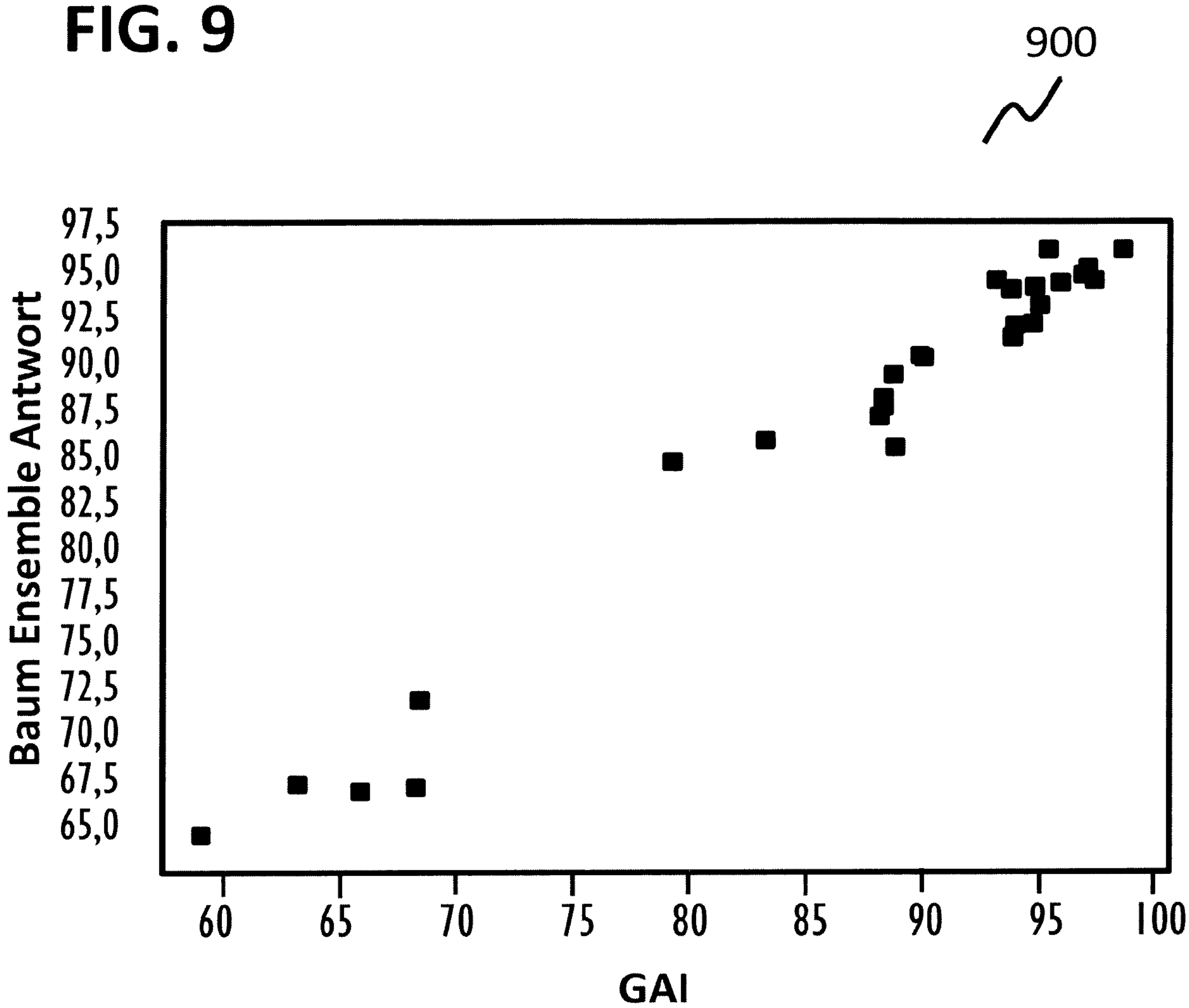
900
Baum Ensemble Antwort
97,5
95,0
92,5
90,0
87,5
85,0
82,5
80,0
77,5
75,0
72,5
70,0
67,5
65,0
60
65
70
75
80
85
90
95
100
GAI

FIG. 10A

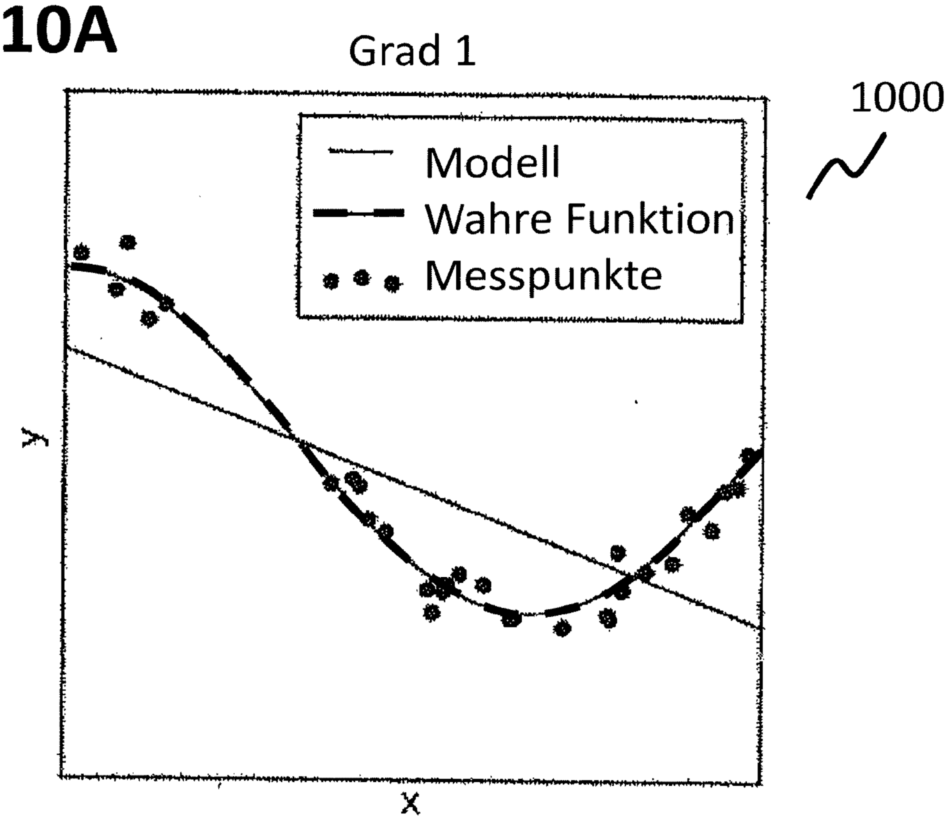


FIG. 10B

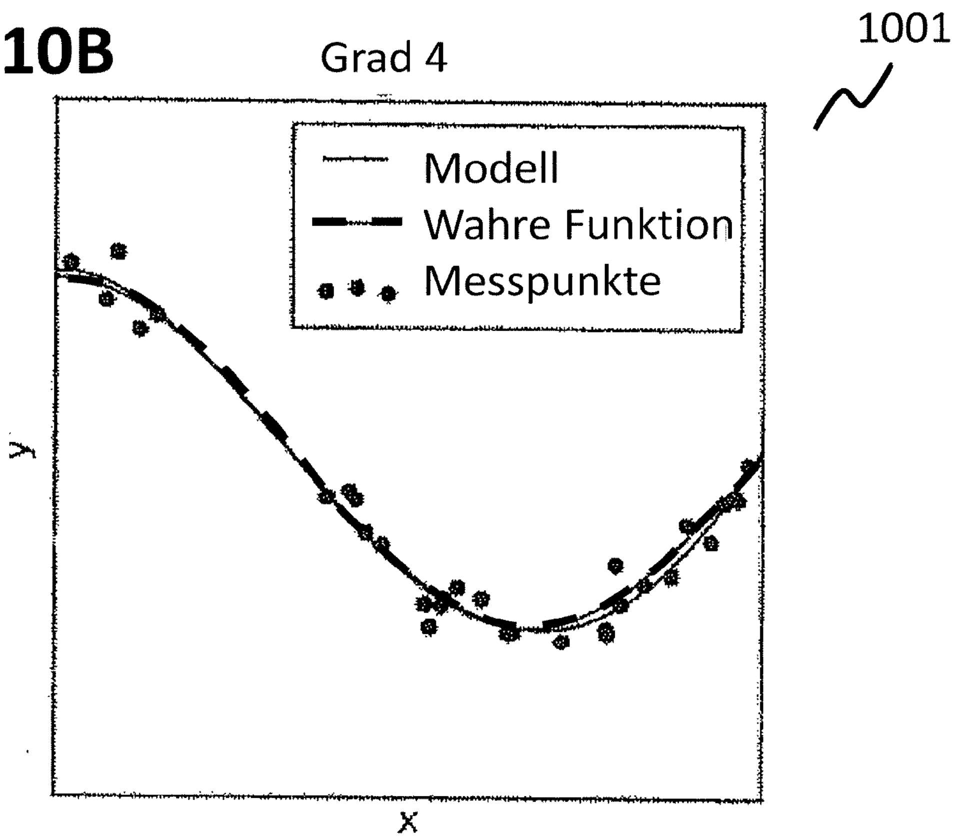

FIG. 10C

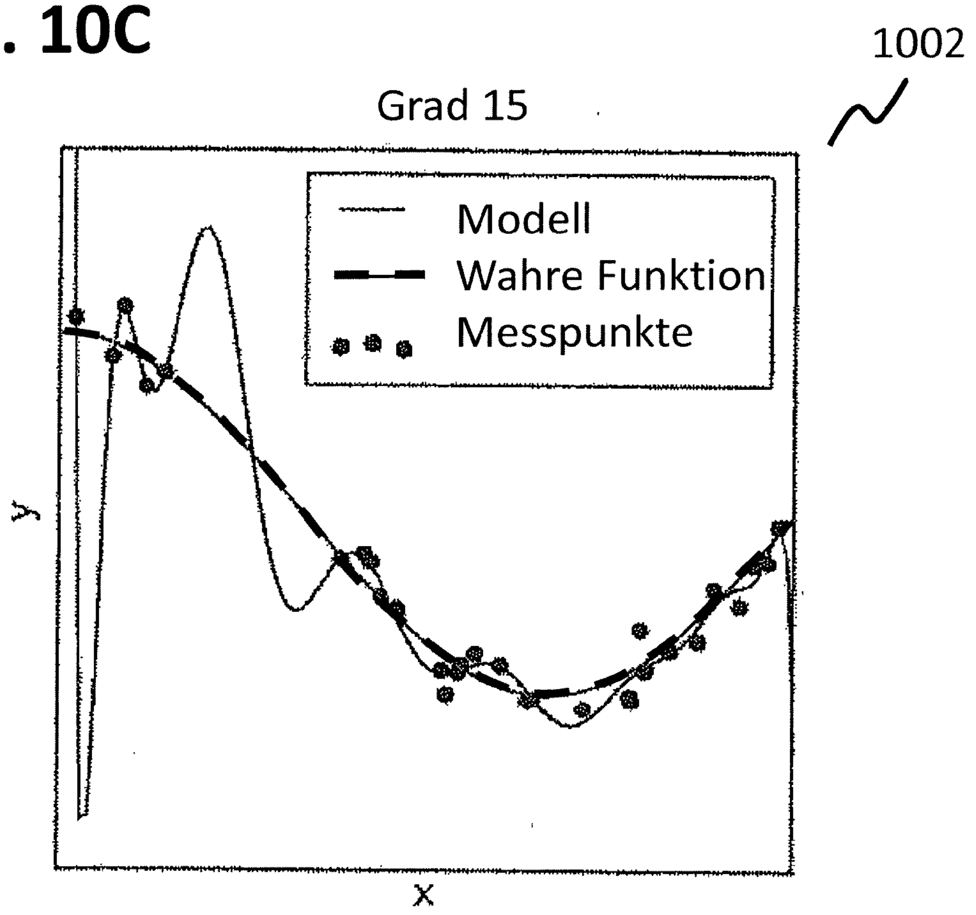

# FIG. 11

1100

1110

Bereitstellen von Haarfarben-Daten, wobei die Haarfarben-Daten für eine Mehrzahl von Färbevorgängen jeweils Werte für eine Mehrzahl von Färbevoraussetzungsparametern und für mindestens einen Färbeergebnisparameter aufweisen, wobei für jeden Färbevorgang der Mehrzahl von Färbevorgängen die Mehrzahl von Färbevoraussetzungsparametern eine erste Konzentration einer ersten Farbstoffvorstufe und eine zweite Konzentration einer zweiten Farbstoffvorstufe aufweist; wobei für jeden Färbevorgang der Mehrzahl von Färbevorgängen der mindestens eine Färbeergebnisparameter eine gemessene Angabe über eine Eigenschaft von Haarfarben aufweist

↓

1120

Ermitteln einer Beziehung zwischen der Mehrzahl von Färbevoraussetzungsparametern und dem mindestens einen Färbeergebnisparameter mittels prädiktiver Analytik basierend auf den Haarfarben-Daten.

FIG. 12

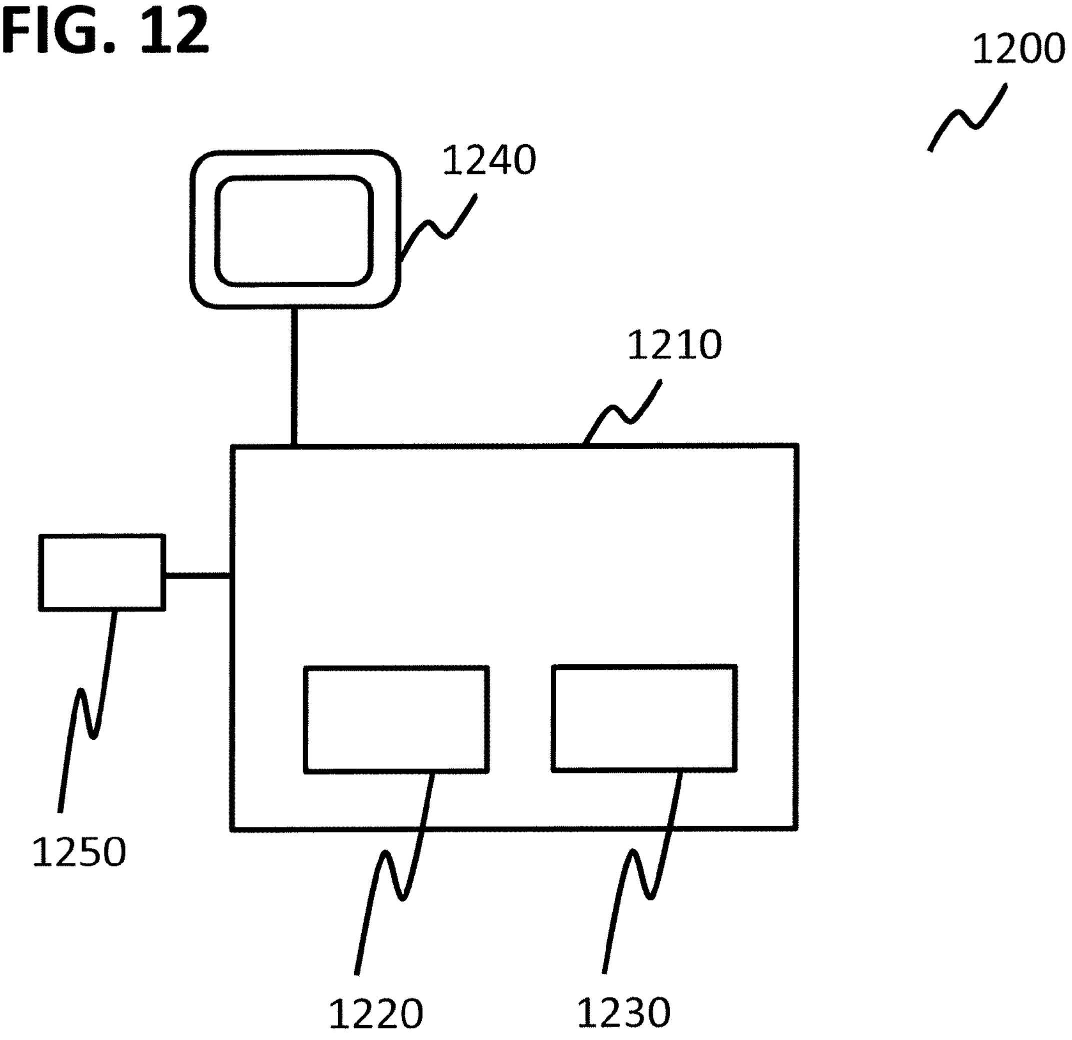

# FIG. 13

1300

1321

| Produkt | dE00(24 W) | p-Toluylen-diaminsulfat [µmol/ 100g] | m-Aminophenol [µmol / 100g] |
|---|---|---|---|
| N&E 542 Mittelaschblond | 2,7 | 859,5 | 130,6 |
| N&E 545 Mittelgoldblond | 1,4 | 1369,6 | 75.5 |
| N&E 550 Dunkelblond | 2,4 | 862,4 | 66.4 |
| N&E 555 Dunkles Goldblond | 2,7 | 089.8 | 72,9 |
| N&E 557 Multi-Reflex-Braun | 1,8 | 1859,6 | 0,0 |
| N&E 550 Hellbraun | 2,8 | 2117,1 | 400.9 |
| N&E 562 Hellaschbraun | 2,9 | 1505,7 | 184.5 |
| M&E 555 Hellgoldbraun | 2,0 | 1811,5 | 166,3 |
| N&E 566 Cinnamon Golden Brown | 1,6 | 3453,3 | 234,0 |
| N&E 568 Intensiv-Rot | 3,4 | 0,0 | 2000,1 |
| N&5 570 Mittelbraun | 1,8 | 2850,1 | 422,7 |
| N&E 574 Zartbitterschokolade | 1,6 | 3861,5 | 0,0 |
| N&E 575 Kastanie Rotbraun | 1,4 | 2810,3 | 304,3 |
| N&E 580 Dunkelbraun | 2,3 | 3758,4 | 989,7 |
| N&E 584 Mokkaschokolade | 1,4 | 3595,9 | 334,8 |
| N&E 585 Multi-Reflex-Braun | 1,9 | 2923,5 | 229,1 |
| N&E 586 Cinnamon Dark Brown | 1,3 | 5616,1 | 0,0 |
| N&E 588 Glossy Acaiberry | 3,1 | 1999,3 | 0,0 |
| N&E 590 Schwarz | 0,5 | 8475,1 | 918,4 |
| Nectra 688 | 2,2 | 0,0 | 2776,8 |
| Nectra 499 | 1,9 | 447,2 | 336,7 |
| Nectra 568 | 1,9 | 1804,3 | 401,7 |
| Nectra 400 | 1,8 | 4133,8 | 1093,5 |
| Syoss Oleo 3-10 | 2,3 | 3759,4 | 938,7 |

1310

1331

1320 1330

Fortsetzung auf nächster Seite

# FIG. 13 (Fortsetzung)

1300

Fortsetzung von vorheriger Seite

| | | | |
|---|---|---|---|
| Syoss Oleo 5-92 | 2,8 | 0,0 | 1963,3 |
| Nectra 320 | 1,1 | 5650,4 | 1484,6 |
| Nectra 468 | 1,4 | 2338,3 | 0,0 |
| Kaschmirrot Variante 2 | 2,0 | 0,0 | 0,0 |
| Syoss Color 2014 5-22 | 2,8 | 491,7 | 2812,0 |
| Syoss Color 2014 1-4 | 2,1 | 4994,3 | 0.0 |
| Syoss Color 2014 5-28 | 1,7 | 1756,7 | 166,0 |
| Syoss Oleo 4-29 | 4,4 | 681,3 | 227,7 |
| Syoss Oleo 1-40 | 2,5 | 4994,3 | 0,0 |
| Igora Royal 5-88 | 2,8 | 481.7 | 2812,0 |
| Nectra 777 | 7,4 | 0,0 | 620,0 |
| Igora Royal 3-0 | 2,2 | 5788.6 | 1512,1 |
| Igora Royal 5-5 | 1,7 | 2043,1 | 165,0 |
| Igora Royal 6-65 | 1,8 | 1793,4 | 183,3 |
| Igora Royal 6-0 | 2,9 | 1922.6 | 440,3 |
| Igora Royal 5-1 | 3,7 | 1566,4 | 247,4 |
| Igora Royal 4-88 | 2,6 | 794.6 | 1374,6 |
| Igora Royal 7-887 | 2,2 | 149.5 | 0,0 |
| Syoss Color 2012 4-2 | 4,1 | 1589,1 | 458,2 |
| Syoss Color 2012 5-22 | 2,8 | 612.9 | 481,1 |
| Syoss Color 2012 1-4 | 2,4 | 4904,3 | 0.0 |
| Syoss Color 2D12 5-29 | 3,2 | 0,0 | 2000,1 |
| Syoss Color 2012 3-55 | 1,3 | 3904,7 | 412,4 |
| Syoss Color 2012 5-0 | 1,9 | 3132.8 | 710,2 |
| Brilliance 880 | 1,5 | 3408.6 | 740,9 |
| Igora Royal 1-0 | 1,0 | 7355.3 | 733,1 |
| Syoss Oleo 2-10 | 1,8 | 5012,5 | 1319,6 |
| Syoss Oleo 4-18 | 2,6 | 2042,8 | 131,5 |
| Syoss Oleo 6-10 | 2,4 | 2144,4 | 163,1 |

1320 1330

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- FR 2984569 B1
- JP 2007212140 A
- JP 2004144569 A
- WO 200187245 A2
- DE 2007050434 A1